(19) Europäisches Patentamt
European Patent Office
Office européen des brevets

(11) EP 4 772 508 A1

(12) EUROPEAN PATENT APPLICATION
published in accordance with Art. 153(4) EPC

(43) Date of publication:
**08.07.2026 Bulletin 2026/28**

(21) Application number: **24858699.2**

(22) Date of filing: **30.08.2024**

(51) International Patent Classification (IPC):
***C07D 471/08*** (2006.01) ***C07D 487/04*** (2006.01)
***C07D 257/00*** (2006.01) ***A61K 51/04*** (2006.01)

(52) Cooperative Patent Classification (CPC):
**A61K 51/04; C07D 257/00; C07D 471/08; C07D 487/04**

(86) International application number:
**PCT/CN2024/115680**

(87) International publication number:
**WO 2025/045168 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.09.2023 CN 202311123501**
**04.02.2024 CN 202410157050**

(71) Applicants:
- **Jiangsu Hengrui Pharmaceuticals Co., Ltd.**
  **Lianyungang, Jiangsu 222047 (CN)**
- **Shanghai Senhui Medicine Co., Ltd.**
  **Shanghai 201203 (CN)**

(72) Inventors:
- **REN, Wenming**
  **Lianyungang, Jiangsu 222047 (CN)**
- **ZHU, Lingjian**
  **Shanghai 201203 (CN)**
- **WANG, Dianru**
  **Shanghai 201203 (CN)**
- **HUANG, Jian**
  **Shanghai 201203 (CN)**
- **LIAO, Cheng**
  **Shanghai 201203 (CN)**

(74) Representative: **Dragotti & Associati S.P.A.**
**Via Nino Bixio, 7**
**20129 Milano (MI) (IT)**

# (54) MACROCYCLIC COMPOUND AND USE THEREOF

(57) The present disclosure relates to a macrocyclic compound and a use thereof. Specifically, the present disclosure provides a compound as shown in formula (I) or a pharmaceutically acceptable salt thereof, wherein $R^1$-$R^3$, $Z^1$-$Z^3$, $K^1$-$K^{12}$, and $X^1$-$X^3$ are as defined in the description.

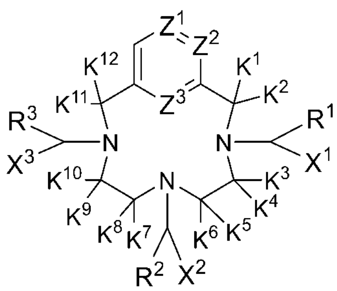

(I)

EP 4 772 508 A1

## Description

## Technical Field

The present disclosure belongs to the field of medicine, and relates to a macrocyclic compound and a use thereof.

## Background Art

Magnetic resonance imaging (MRI) is a medical imaging technique that generates images by reconstructing signals emitted from atomic nuclei (such as hydrogen nuclei) in a magnetic field. The MRI signal depends on the environment surrounding the visualized nuclei and their longitudinal and transverse relaxation times, referred to as T1 and T2, respectively. Therefore, when the visualized nuclei are protons, the MRI signal intensity will depend on factors such as proton density and the chemical environment of the protons.

Contrast agents containing paramagnetic metal ions can be used in MRI to improve imaging contrast, including gadolinium complex compounds characterised by high stability constants which can ensure the prevention of free metal ions (known as being highly toxic to living organisms) release in vivo.

For example, WO 2007042506 discloses a PCTA derivative of the following formula, wherein D represents CH or N, E represents CH or N, F1 represents CH or N, and others are as defined in the text:

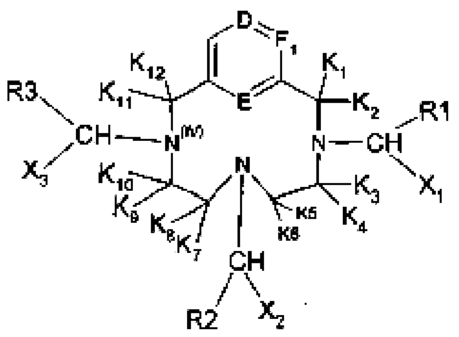

In addition, other types of contrast agents containing paramagnetic metal ions have been reported in, such as WO 1993011800, WO 2000075141, WO 2003074523, EP 0438206, WO 2006100305, WO 2018115314, WO 2019122255, US 5403572, EP 0661279, US 20200353105 and US 5334371.

## Summary of the Invention

The present disclosure provides a compound as shown in formula I or a pharmaceutically acceptable salt thereof,

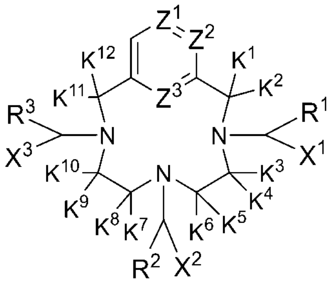

wherein $R^1$, $R^2$ and $R^3$ are each independently selected from -COOH, - $P(O)(OH)_2$ or $-P(O)(R^4)(OH)$, and $R^4$ is selected from hydrogen or $C_{1-4}$ alkyl;

$X^1$, $X^2$ and $X^3$ are each independently selected from L-Y, wherein L is selected from substituted or unsubstituted $C_{1-6}$ alkylene, and Y is selected from - COOH, $-CONH_2$, $-CONR^5R^6$ or $-NR^7COR^8$;

$R^5$ is selected from hydrogen, $C_{1-6}$ alkyl or $C_{1-6}$ hydroxylalkyl, $R^6$ is selected from $C_{1-6}$ alkyl or $C_{1-6}$ hydroxylalkyl, and at least one of $R^5$ and $R^6$ is $C_{1-6}$ hydroxyalkyl;

$R^7$ is selected from hydrogen, $C_{1-6}$ alkyl or $C_{1-6}$ hydroxylalkyl, $R^8$ is selected from $C_{1-6}$ alkyl or $C_{1-6}$ hydroxylalkyl, and at least one of $R^7$ and $R^8$ is $C_{1-6}$ hydroxyalkyl;

$Z^1$ is $CR^9$;
$Z^2$ is $CR^{10}$;
$R^9$ and $R^{10}$ are independently selected from hydrogen, halogen, nitro, amino, cyano, $C_{1-6}$ alkyl, $C_{1-6}$ alkoxy, 3- to 6-membered cycloalkyl, 3- to 6-membered cycloalkoxy, 3- to 6-membered heterocycloalkyl, 3- to 6-membered heterocycloalkoxy, 6-membered aryl or 5- to 6-membered heteroaryl, wherein the alkyl, alkoxy, cycloalkyl, heterocycloalkyl, cycloalkoxy, heterocyclyloxy, aryl or heteroaryl is optionally substituted with one or more $R^{1A}$, each of which is independently selected from halogen, hydroxyl, oxo, nitro, cyano, amino, $C_{1-6}$ alkyl or $C_{1-6}$ alkoxy, and $R^9$ and $R^{10}$ are not both hydrogen;
or $R^9$ and $R^{10}$, together with adjacent carbon atoms, form 6-membered aryl or 5- to 6-membered heteroaryl, wherein the aryl or heteroaryl is optionally substituted with one or more $R^{1B}$, each of which independently selected from halogen, hydroxyl, nitro, cyano, amino, substituted or unsubstituted $C_{1-6}$ alkyl, or substituted or unsubstituted $C_{1-6}$ alkoxy;
$Z^3$ is selected from CH or N;
$K^1$-$K^{12}$ are independently selected from hydrogen, $C_{1-6}$ alkyl or $C_{1-6}$ hydroxylalkyl;
or $K^1$ and $K^2$, $K^3$ or $K^4$ and $K^5$ or $K^6$, $K^7$ or $K^8$ and $K^9$ or $K^{10}$, or $K^{11}$ and $K^{12}$ form a 3- to 6-membered carbocyclic ring.

In some embodiments, in the compound as shown in formula I or the pharmaceutically acceptable salt thereof, $K^1$ and $K^2$ form a 3- to 6-membered substituted or unsubstituted carbocyclic ring, or $K^3$ or $K^4$ forms a 3- to 6-membered substituted or unsubstituted carbocyclic ring with $K^5$ or $K^6$, or $K^7$ or $K^8$ forms a 3- to 6-membered substituted or unsubstituted carbocyclic ring with $K^9$ or $K^{10}$, or $K^{11}$ and $K^{12}$ form a 3- to 6-membered substituted or unsubstituted carbocyclic ring.

In some embodiments, in the compound as shown in formula I or the pharmaceutically acceptable salt thereof, $R^1$, $R^2$ and $R^3$ are -COOH.

In some embodiments, in the compound as shown in formula I or the pharmaceutically acceptable salt thereof, $R^1$, $R^2$ and $R^3$ are $-P(O)(OH)_2$.

In some embodiments, in the compound as shown in formula I or the pharmaceutically acceptable salt thereof, $R^1$, $R^2$ and $R^3$ are $-P(O)(R^4)(OH)$, wherein $R^4$ is selected from hydrogen or $C_{1-4}$ alkyl (such as, methyl or ethyl).

In other embodiments, in the compound as shown in formula I or the pharmaceutically acceptable salt thereof, $Z^3$ is N.

In some embodiments, in the compound as shown in formula I or the pharmaceutically acceptable salt thereof, $Z^1$ is $CR^9$, and $R^9$ is selected from halogen, $C_{1-6}$ alkyl, $C_{1-6}$ alkoxy, 6-membered aryl or 5- to 6-membered heteroaryl, wherein the alkyl, alkoxy, aryl or heteroaryl is optionally substituted with one or more $R^{1A}$ as defined above.

In some embodiments, in the compound as shown in formula I or the pharmaceutically acceptable salt thereof, $R^9$ is selected from hydrogen, chlorine, fluorine, methoxy, ethoxy or phenyl.

In some embodiments, the compound as shown in formula I or the pharmaceutically acceptable salt thereof as provided is a compound as shown in formula II-1 or a pharmaceutically acceptable salt thereof,

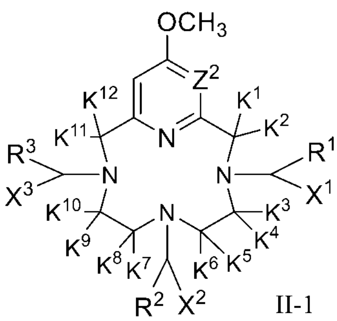

II-1

wherein $R^1$-$R^3$, $Z^2$, $K^1$-$K^{12}$, and $X^1$-$X^3$ are as defined above.

In some embodiments, in the compound as shown in formula I or the pharmaceutically acceptable salt thereof, $Z^2$ is $CR^{10}$, and $R^{10}$ is selected from hydrogen, halogen, $C_{1-6}$ alkyl, $C_{1-6}$ alkoxy, 6-membered aryl or 5- to 6-membered heteroaryl, wherein the alkyl, alkoxy, aryl or heteroaryl is optionally substituted with one or more $R^{1A}$ as defined above.

In some embodiments, in the compound as shown in formula I or the pharmaceutically acceptable salt thereof, $R^{10}$ is selected from hydrogen, chlorine, fluorine, methoxy, ethoxy or phenyl.

In addition, in some embodiments, in the compound as shown in formula I or the pharmaceutically acceptable salt thereof, $R^9$ and $R^{10}$, together with adjacent carbon atoms, form 6-membered aryl, wherein the aryl is optionally substituted with one or more $R^{1B}$ as defined above.

In other embodiments, the compound of formula I is

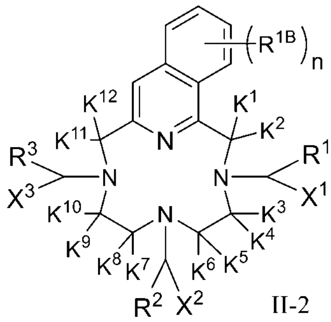

II-2 ,

wherein n is an integer from 0 to 4; $R^1$-$R^3$, $K^1$-$K^{12}$, $X^1$-$X^3$ and $R^{1B}$ are as defined in the compound as shown in formula I.

In some embodiments, in the compound as shown in formula I or formula II or the pharmaceutically acceptable salt thereof as provided, $X^1$, $X^2$ and $X^3$ are each independently selected from L-Y, wherein L is selected from substituted or unsubstituted $C_{1-3}$ alkylene, and Y is selected from -COOH or -$CONH_2$.

In other embodiments, in the compound as shown in formula I or formula II or the pharmaceutically acceptable salt thereof as provided, $X^1$, $X^2$ and $X^3$ are each independently selected from L-Y, wherein L is selected from substituted or unsubstituted $C_{1-3}$ alkylene, and Y is selected from -$CONR^5R^6$ or -$NR^7COR^8$, $R^5$ is selected from hydrogen or methyl, $R^6$ is $C_{1-6}$ hydroxylalkyl, $R^7$ is selected from hydrogen or methyl, and $R^8$ is $C_{1-6}$ hydroxylalkyl.

In other embodiments, in the compound as shown in formula I or formula II or the pharmaceutically acceptable salt thereof, $R^6$ is $C_{2-6}$ hydroxylalkyl, including, but not limited to, -$CH_2CH_2OH$, -$CH(OH)CH_2OH$, -$CH(CH_2OH)_2$, - $CH_2(CHOH)_pCH_2OH$ or -$C(CH_2OH)_3$, wherein p is an integer from 1 to 4.

In some embodiments, in the compound as shown in formula I or the pharmaceutically acceptable salt thereof, $R^8$ is $C_{2-6}$ hydroxylalkyl, including, but not limited to, -$CH_2CH_2OH$, -$CH(OH)CH_2OH$, -$CH(CH_2OH)_2$, - $CH_2(CHOH)_pCH_2OH$ or -$C(CH_2OH)_3$, wherein p is an integer from 1 to 4.

In some embodiments, in the compound as shown in formula I or the pharmaceutically acceptable salt thereof, $X^1$, $X^2$ and $X^3$ are each independently selected from L-Y, wherein L is selected from substituted or unsubstituted $C_{1-3}$ alkylene, and Y is -$CONR^5R^6$, $R^5$ is hydrogen, $R^6$ is selected from -$CH_2CH_2OH$, - $CH(OH)CH_2OH$, -$CH(CH_2OH)_2$, -$CH_2(CHOH)_pCH_2OH$ or -$C(CH_2OH)_3$, and p is an integer from 1 to 4.

The present disclosure further provides an isotopic substitute of the aforementioned compound or the pharmaceutically acceptable salt thereof. In some embodiments, the isotopic substitute is a deuterated compound.

The present disclosure further provides a multimer of the aforementioned compound or the pharmaceutically acceptable salt thereof. In some embodiments, the multimer is a dimer or trimer. In some embodiments, the compounds as shown in formula I or formula II are coupled together via a bonding group. For example, the bonding group is bonded to the compound as shown in formula I or formula II at an aromatic or heteroaromatic ring.

The present disclosure also provides a carrier compound, which comprises the aforementioned compound or the isotopic substitute thereof coupled to a biological carrier via an optional intermediary bonding group. In some embodiments, the biological carrier is targeted to a specific pathological area.

In other embodiments, the bonding group may be bonded to the compound as shown in formula I at an aromatic or heteroaromatic ring. In other embodiments, in the absence of a bonding group, the biological carrier may be bonded to the compound as shown in formula I at $X^1$, $X^2$ or $X^3$.

For example, WO 2004112839 discloses various biological carriers that can be used, particularly the carrier compounds illustrated on pages 135 to 137. For the purpose of illustrating the carrier compounds of the present disclosure, WO 2004112839 is incorporated herein by reference for illustration.

WO 2005/049005, WO 2005/049095, WO 2005/042033, and WO 2001/9188 also disclose other biological carriers, such as biological carriers targeting VEGF and angiopoietin receptors, fibrin-targeting polypeptides, integrin-targeting peptides and metalloproteinase-targeting peptides.

In another aspect, bonding groups include, but are not limited to: A) -$(CH_2)_2$-phenyl-NH, -$(CH_2)_3$-NH, -NH-$(CH_2)_2$-NH, absence, or a single bond;

B) $P_1$-$L_1$-$P_2$, wherein $P_1$ and $P_2$ are each independently selected from O, S, NH, -$CO_2$, -NCS, -NCO, -$SO_3H$, -NHCO, -CONH, or absence, and $L_1$ is $C_{1-6}$ alkylene.

The present disclosure also provides a complex of the aforementioned compound or isotopic substitute or multimer or carrier compound and M, wherein M is $Gd^{3+}$, $Mn^{2+}$ or $Fe^{3+}$.

Typical complexes include, but are not limited to:

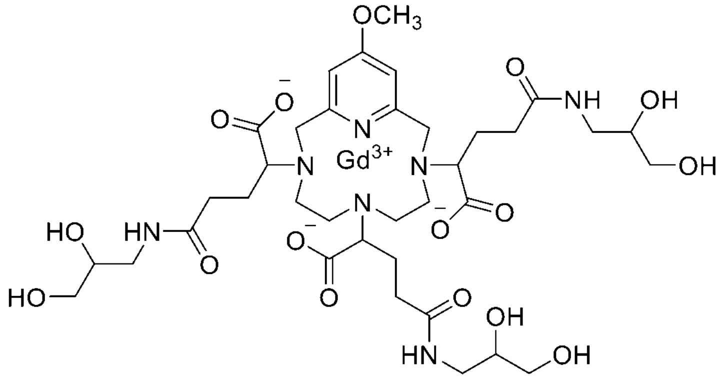

,

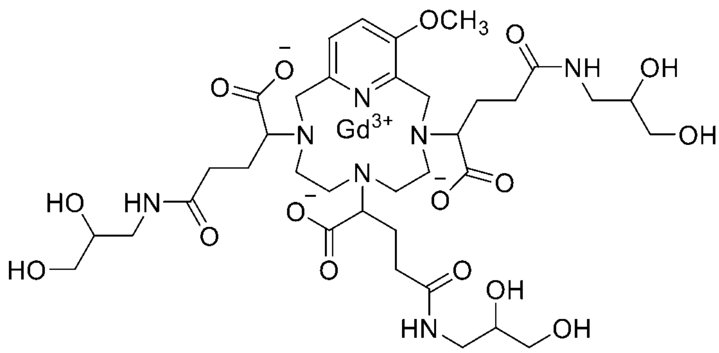

,

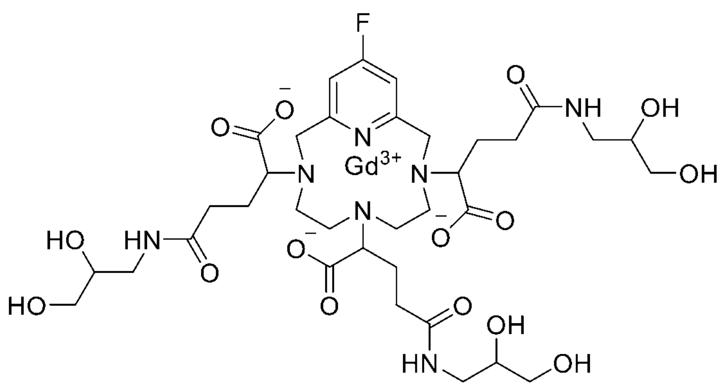

,

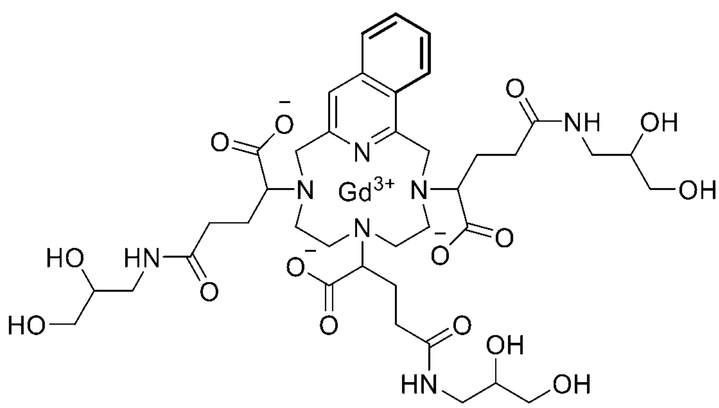

,

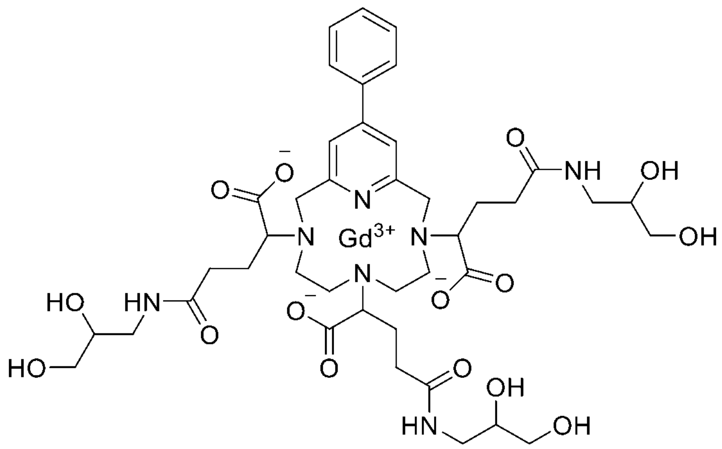

,

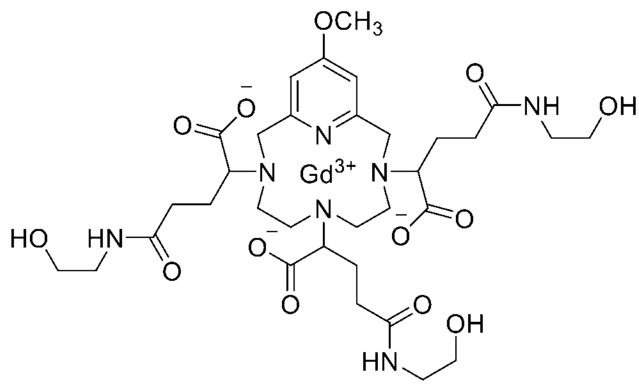

,

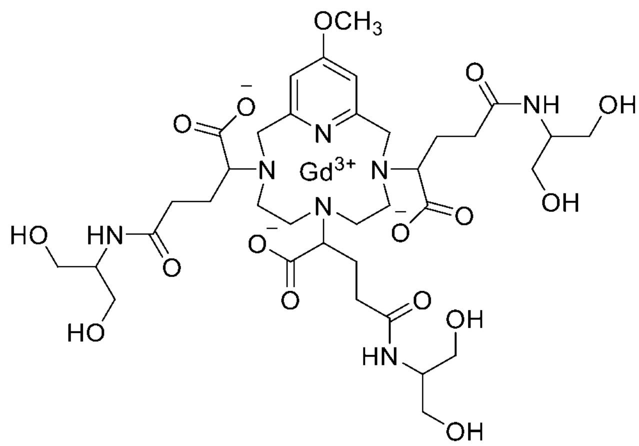

,

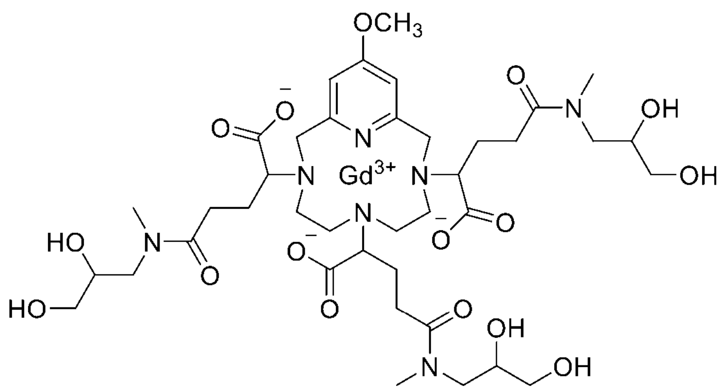

or

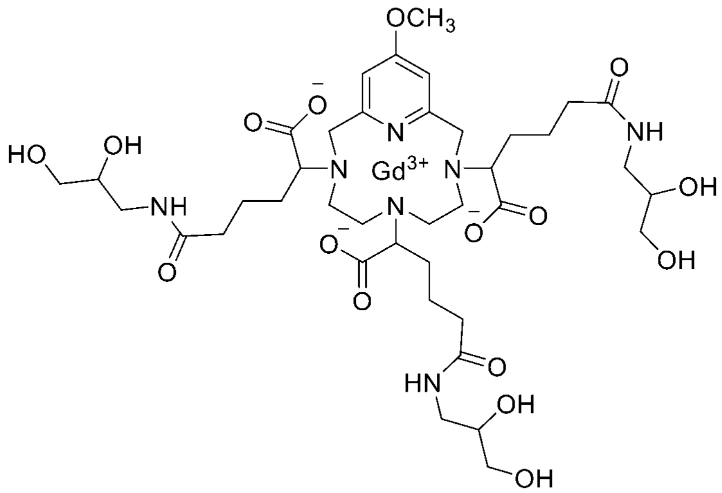

.

In another aspect, the empty orbitals of gadolinium accept lone pairs of electrons donated by N atoms in the macrocycle, forming a structure similar to the one with following formula:

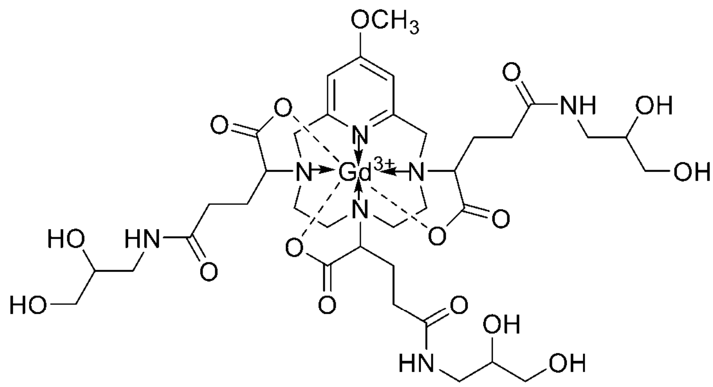

or

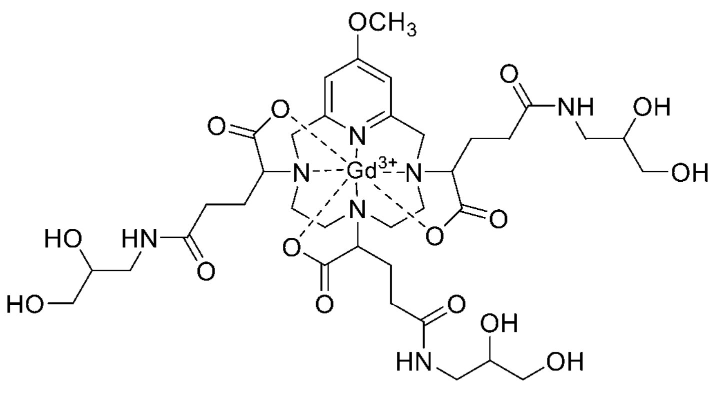

.

In some embodiments, the complex as provided has a structure as follows:

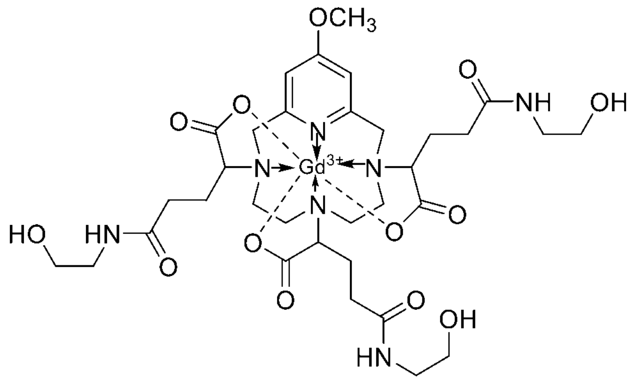

,

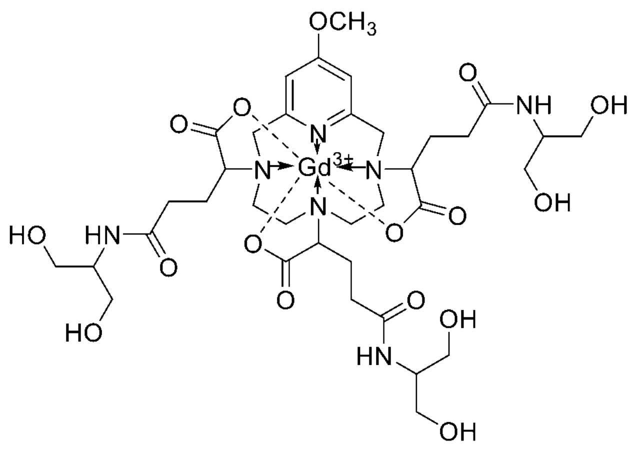

,

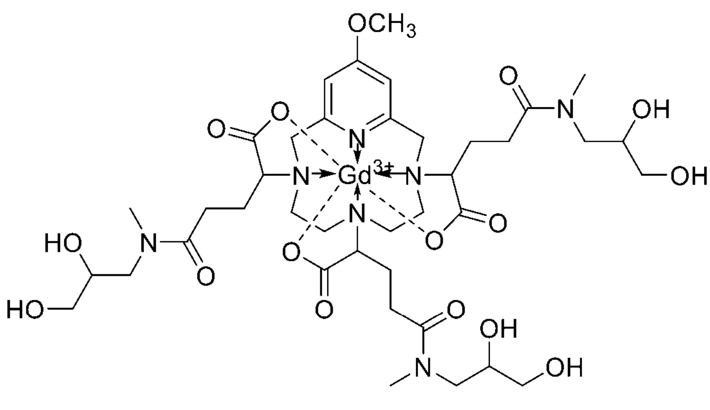

or

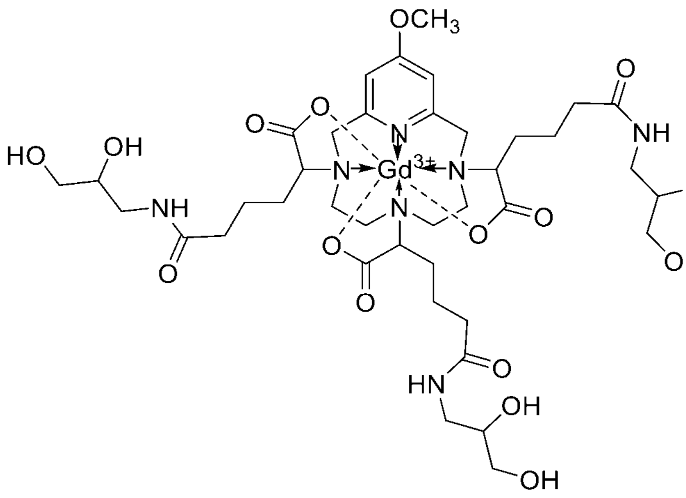

.

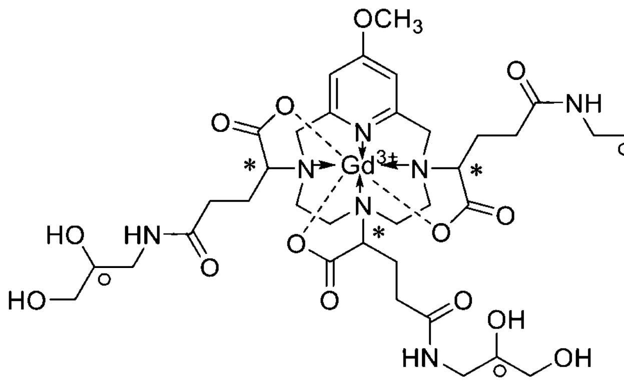

Compound 4

comprises 6 stereocentres (marked with asterisks and open circles respectively in the structure), resulting in a total of 64 stereoisomers.

In some embodiments, compound 4 includes (without considering the chirality of isoserinol):

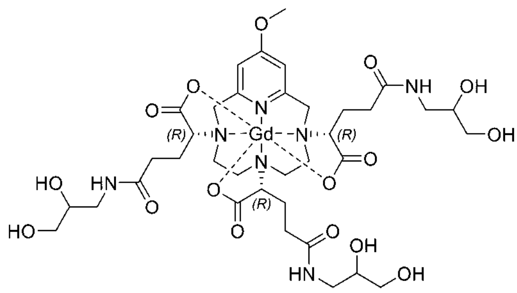

,

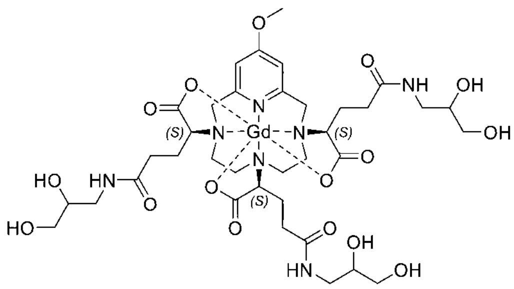

,

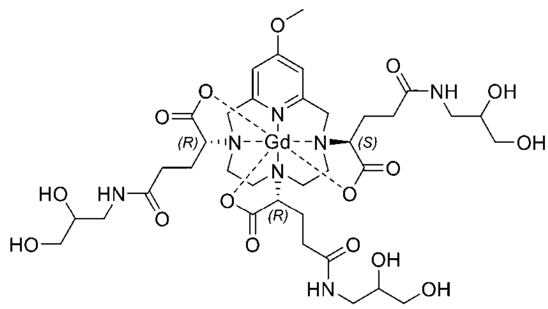

,

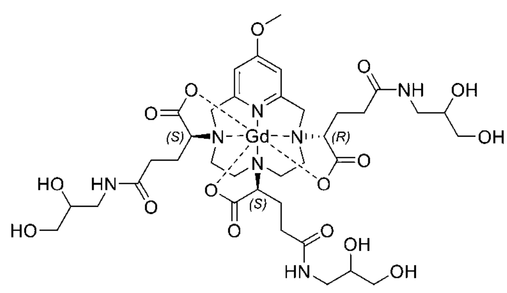

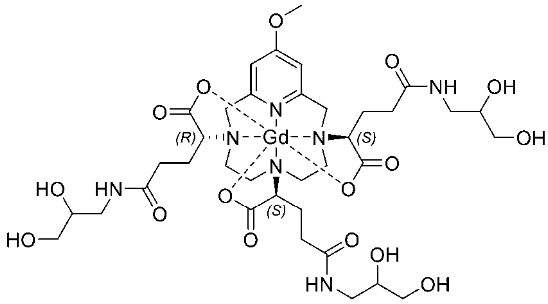,

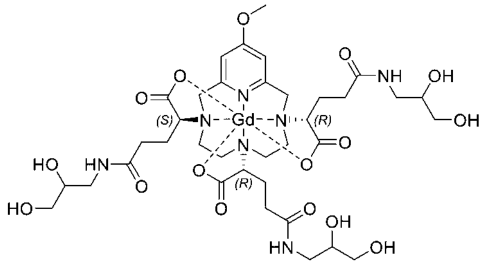,

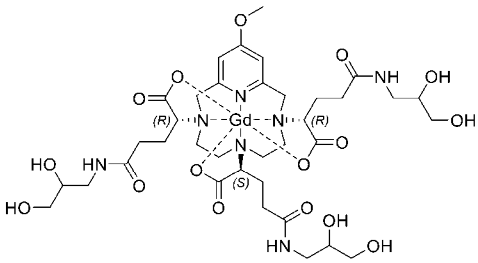, or

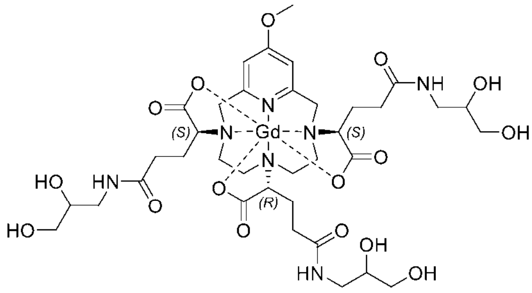

.

The present disclosure also provides a pharmaceutical composition, comprising at least one therapeutically effective amount of the aforementioned compound as shown in formula I or the pharmaceutically acceptable salt thereof, or isotopic substitute thereof, or multimer thereof, or carrier compound thereof, or complex thereof and a pharmaceutically acceptable excipient.

In some embodiments, the pharmaceutical composition comprises the following compound or a pharmaceutically acceptable salt thereof, and a pharmaceutically acceptable excipient

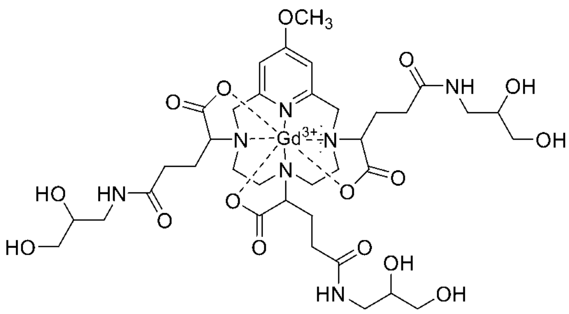

.

In some embodiments, the pharmaceutical composition comprises a RRR/SSS isomer

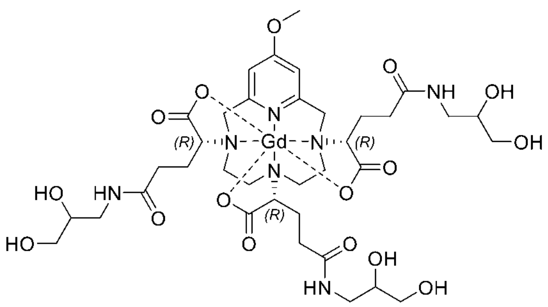

/

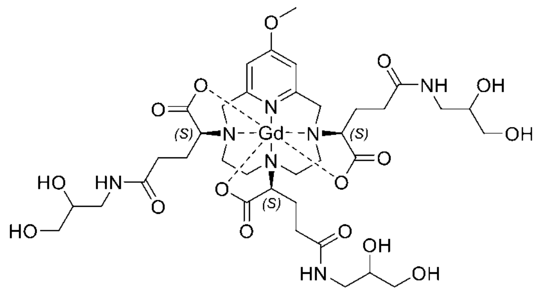

,

and a pharmaceutically acceptable excipient.

In some embodiments, the pharmaceutical composition comprises a RRS/SSR isomer

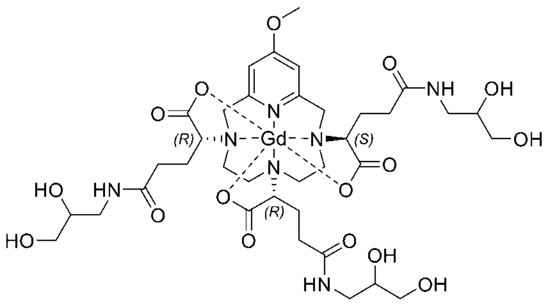

/

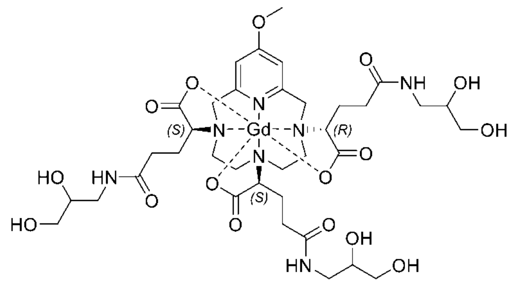

,

and a pharmaceutically acceptable excipient.

In some embodiments, the pharmaceutical composition comprises a RSS/SRR isomer

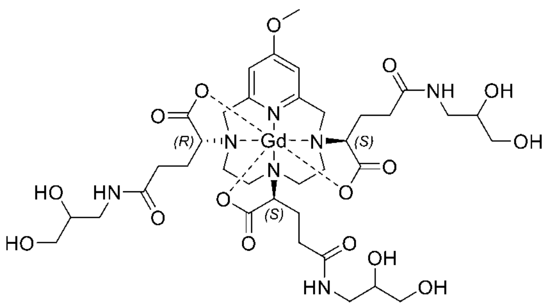

/

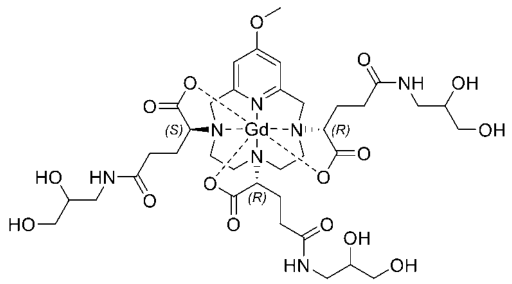

,

and a pharmaceutically acceptable excipient.

In some embodiments, the pharmaceutical composition comprises a RSR/SRS isomer

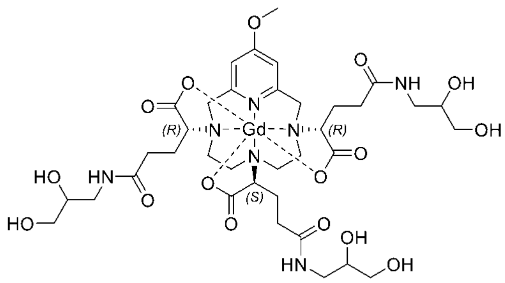

/

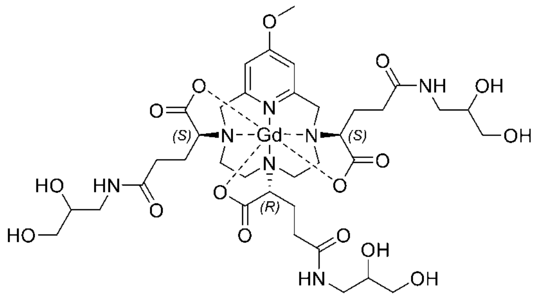

,

and a pharmaceutically acceptable excipient.

In some embodiments, the pharmaceutical composition comprises the following compound or a pharmaceutically acceptable salt thereof, and a pharmaceutically acceptable excipient

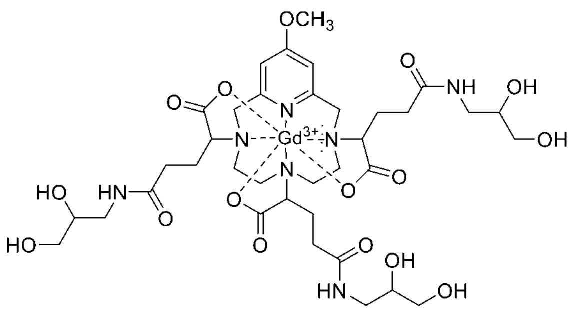

,

wherein at least 50% of the compound is the RRR/SSS isomer

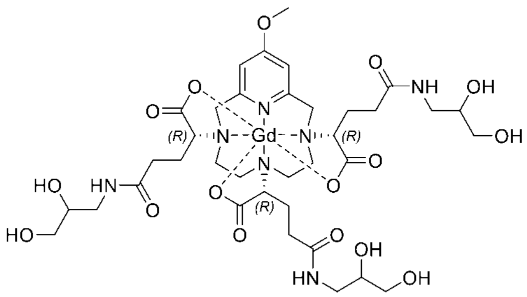

/

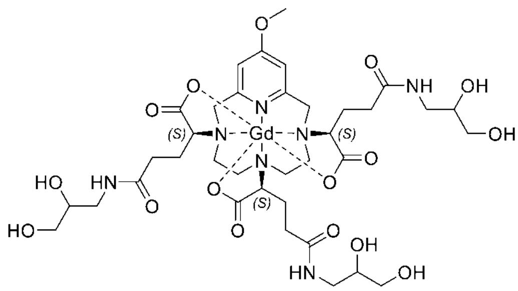

.

In some embodiments, the pharmaceutical composition comprises the following compound or a pharmaceutically acceptable salt thereof, and a pharmaceutically acceptable excipient

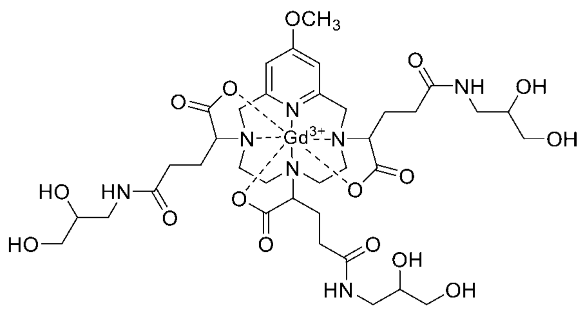

,

wherein at least 70% of the compound is the RRR/SSS isomer

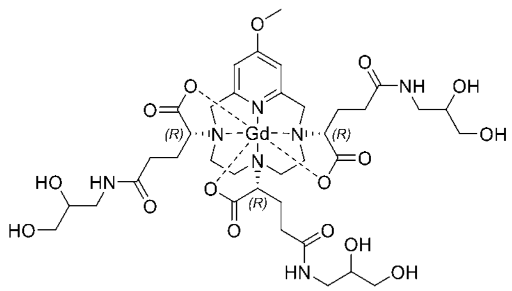

/

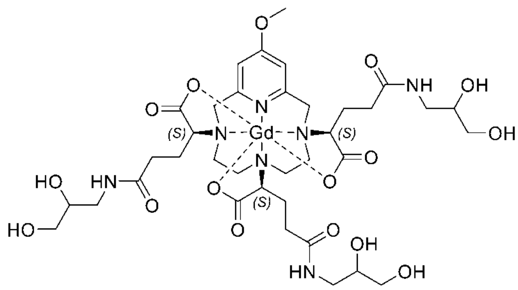

.

In some embodiments, the pharmaceutical composition comprises the following compound or a pharmaceutically acceptable salt thereof, and a pharmaceutically acceptable excipient

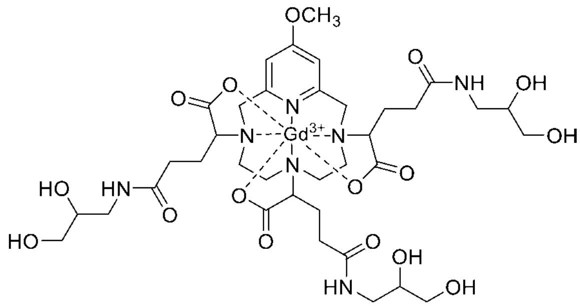

,

wherein at least 90% of the compound is the RRR/SSS isomer

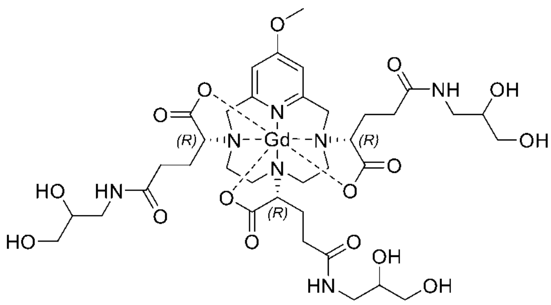

/

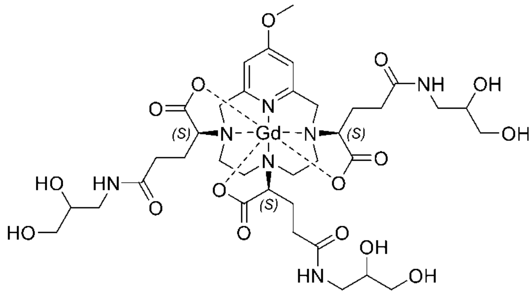

.

In some embodiments, the content of the RRR/SSS isomer

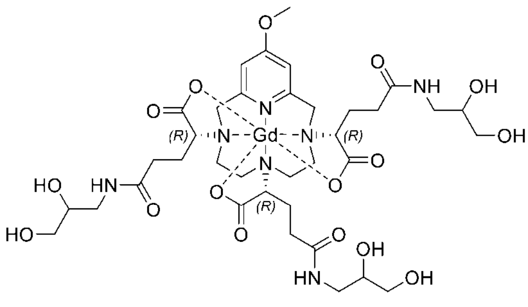

/

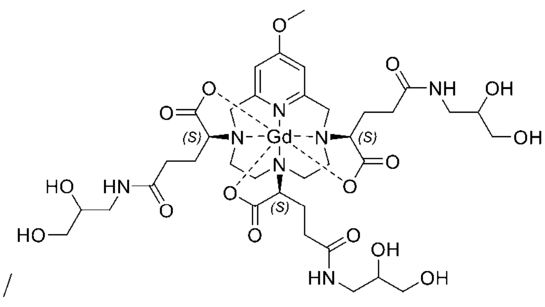

in the pharmaceutical composition is at least 90%, such as 91%, 92%, 93%, 94%, 95%, 96%, 97%, 98%, 99% or higher (e.g., 100%).

The present disclosure also provides the following compound or a salt thereof

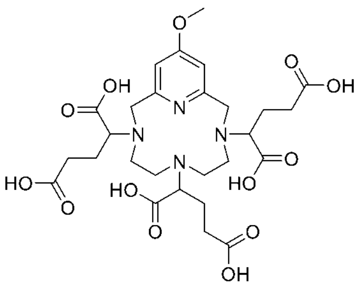

, or

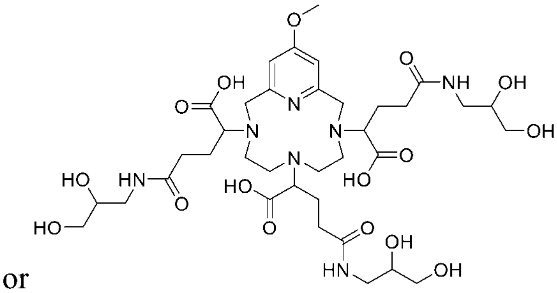

.

In some embodiments, the unit dose of the pharmaceutical composition is 0.001 mg-1000 mg.

In certain embodiments, the pharmaceutical composition comprises 0.01%-99.99% of the aforementioned compound or the pharmaceutically acceptable salt thereof, or isotopic substitute thereof, or multimer thereof, or carrier compound thereof, or complex thereof, on the basis of the total weight of the composition. In certain embodiments, the pharmaceutical composition comprises 0.1%-99.9% of the aforementioned compound or the pharmaceutically acceptable salt thereof, or isotopic substitute thereof, or multimer thereof, or carrier compound thereof, or complex thereof. In certain embodiments, the pharmaceutical composition comprises 0.5%-99.5% of the aforementioned compound or the pharmaceutically acceptable salt thereof, or isotopic substitute thereof, or multimer thereof, or carrier compound thereof, or complex thereof. In certain embodiments, the pharmaceutical composition comprises 1%-99% of the aforementioned compound or the pharmaceutically acceptable salt thereof, or isotopic substitute thereof. In certain embodiments, the pharmaceutical composition comprises 2%-98% of the aforementioned compound or the pharmaceutically acceptable salt thereof, or isotopic substitute thereof, or multimer thereof, or carrier compound thereof, or complex thereof.

In certain embodiments, the pharmaceutical composition comprises 0.01%-99.99% of the pharmaceutically acceptable excipient, on the basis of the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The present disclosure also provides the use of the aforementioned compound as shown in formula I or the pharmaceutically acceptable salt thereof, or isotopic substitute thereof, or multimer thereof, or carrier compound thereof, or complex thereof, or pharmaceutical composition thereof in the preparation of a drug for nuclear magnetic resonance imaging.

The present disclosure also provides the aforementioned compound as shown in formula I or the pharmaceutically acceptable salt thereof, or isotopic substitute thereof, or multimer thereof, or carrier compound thereof, or complex thereof, or pharmaceutical composition thereof for nuclear magnetic resonance imaging.

The present disclosure also provides a method for nuclear magnetic resonance imaging, wherein the method comprises administering to a subject an effective amount of the aforementioned compound as shown in formula I or the pharmaceutically acceptable salt thereof, or isotopic substitute thereof, or multimer thereof, or carrier compound thereof, or complex thereof, or pharmaceutical composition thereof.

The pharmaceutically acceptable salts of the compounds described in the present disclosure may be selected from inorganic salts or organic salts.

In another aspect, the present disclosure also provides a method for preparing compound 4 or a salt thereof

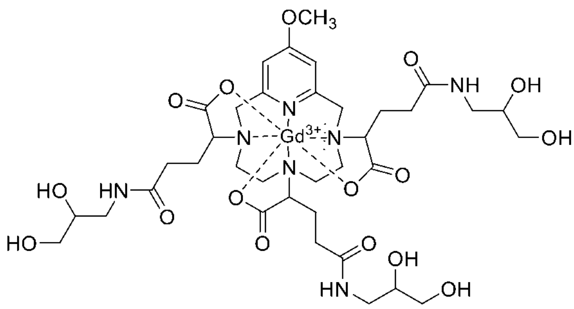

,

wherein the method comprises the steps of reacting compound A with compound B to form the compound 4,

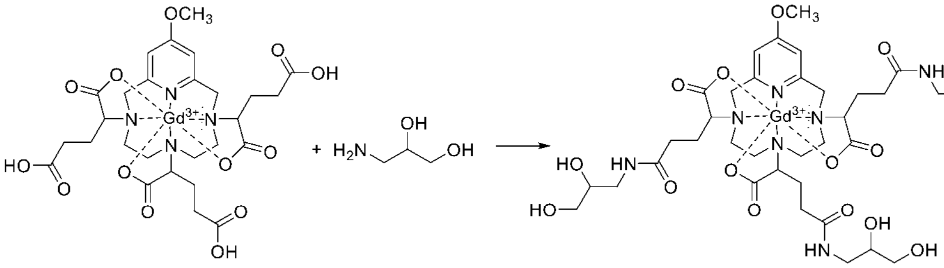

A B

In some embodiments, the compound A is reacted with the compound B in the presence of a condensation reagent, wherein the condensation reagent is selected from, but is not limited to, 1-hydroxybenzotriazole (HOBT) or 1-ethyl-(3-dimethylaminopropyl)carbodiimide (EDCI).

The present disclosure also provides compound A or a salt thereof

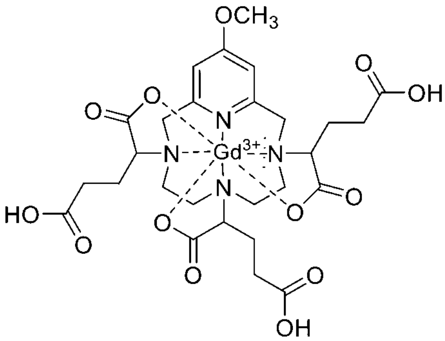

A

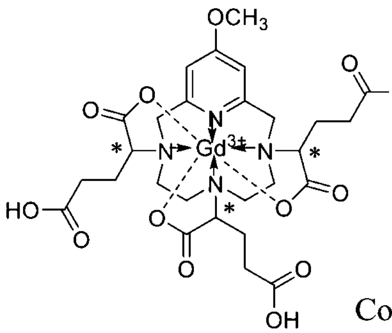

Compound A

comprises 3 stereocentres (marked with asterisks respectively in the structure), resulting in a total of 8 stereoisomers, as shown in the following table:

| | |
|---|---|
| RRR | SSS |
| RSR | SRS |
| RRS | SSR |
| RSS | SRR |

Isomer RRR and isomer SSS are mirror images of each other and are referred to as enantiomers. The enantiomers exhibit the same physicochemical properties, such as melting point, water solubility, and relaxivity. Except for isomers RRR and SSS, the other six isomers are referred to as diastereomers. In an embodiment, a preparative HPLC method is used, wherein four signal peaks are isolated from the compound A obtained by non-stereoselective synthesis, corresponding to the four pairs of enantiomers formed by the different optical isomers listed in the preceding table.

In another aspect, the present disclosure relates to the enrichment of the aforementioned enantiomers or mixtures thereof. "Enrichment" refers to the isomers, or enantiomers, or pairs of enantiomers of the present disclosure that

is obtained with a higher content via existing techniques such as stereoselective synthesis. For the stereoselective synthesis method, reference is made to CN 112533921A, the relevant content of which is incorporated herein by reference for illustration.

In another aspect, the enrichment method comprises obtaining the compound via preparative HPLC.

In some embodiments, the enrichment level of the RRR/SSS isomer of

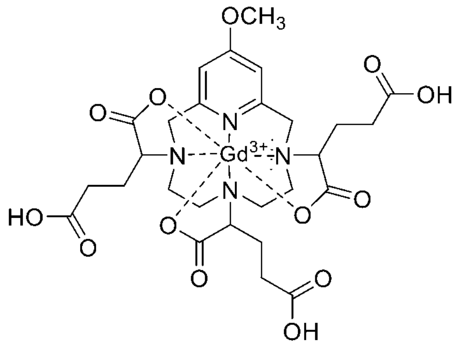

A

is at least 50%, such as 60%, 65%, 70%, 75%, 80%, 85%, 90%, 95% or higher.

In some embodiments, the enriched RRR/SSS enantiomer pair of compound A is reacted with isoserinol to form the RRR/SSS isomers

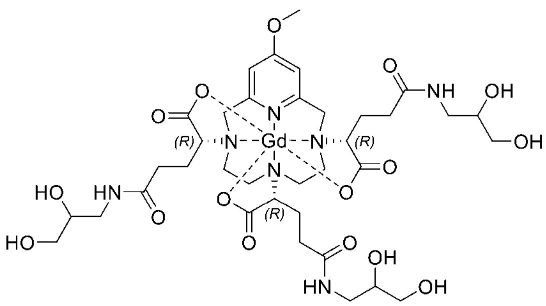

/

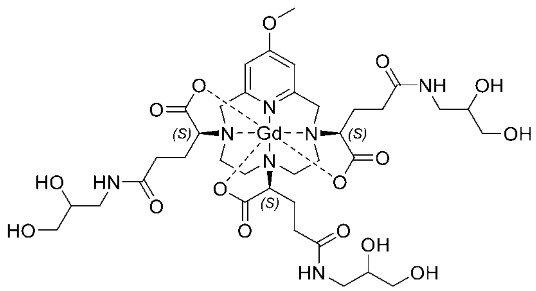

of compound 4.

In some embodiments, the enriched RRS/SSR enantiomer pair of compound A is reacted with isoserinol to form the RRS/SSR isomers

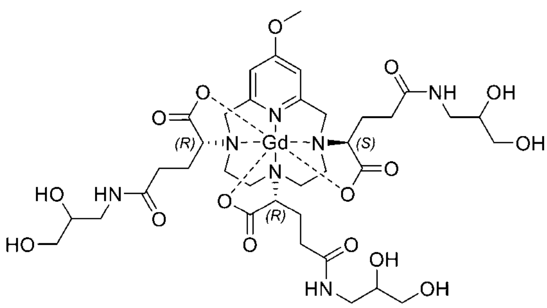

/

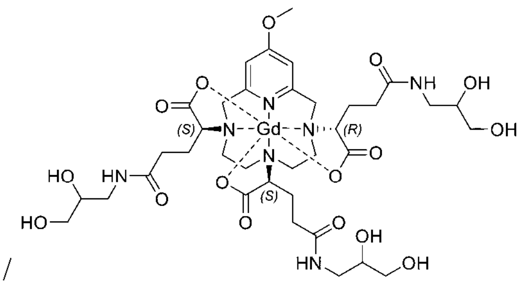

of compound 4.

In some embodiments, the enriched RSS/SRR enantiomer pair of compound A is reacted with isoserinol to form the RSS/SRR isomers

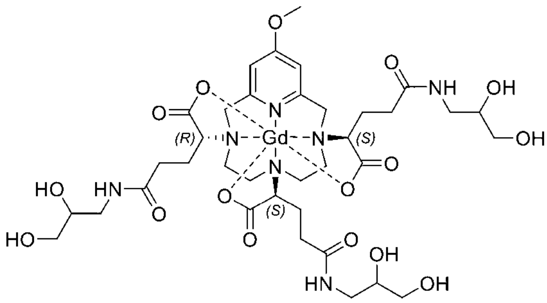

/

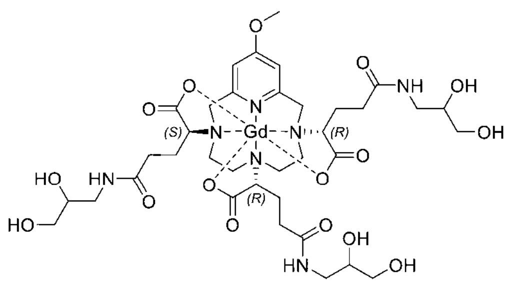

of compound 4.

In some embodiments, the enriched RSR/SRS enantiomer pair of compound A is reacted with isoserinol to form the RSR/SRS isomers

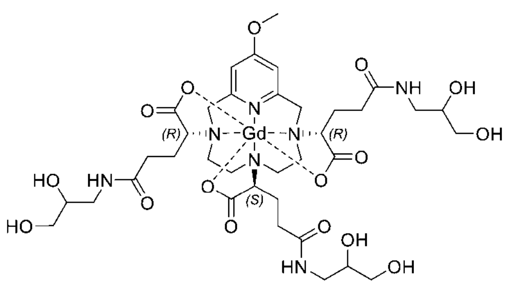

/

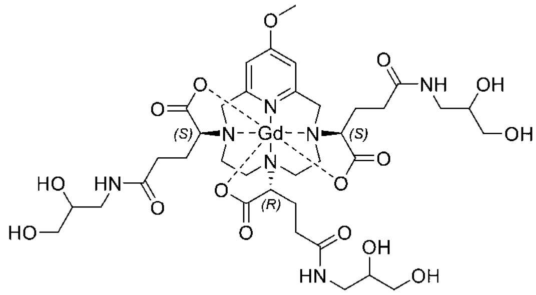

of compound 4.

In other embodiments, in the longitudinal relaxivity r1 test experiment in human plasma (the longitudinal relaxation time T1 is measured at a 0.5 T magnetic field strength using a medium-sized nuclear magnetic resonance imaging analyser (MesoMR23-060-I), and the relaxivity $r_1$ is calculated based on the relaxation time T1 of samples at different concentrations according to the relaxivity calculation formula $r = \frac{1}{\Delta T}/C$ ), the relaxivity of the aforementioned RRR/SSS isomer

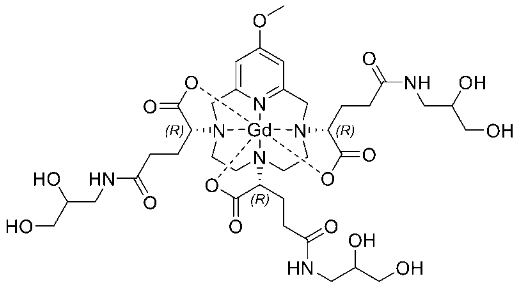

/

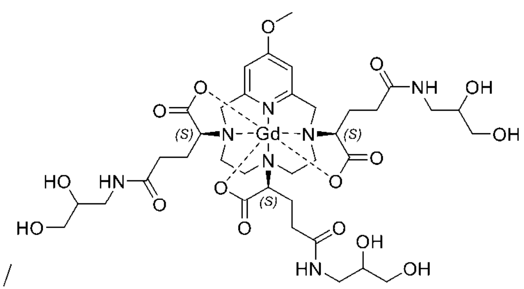

is 21.8 $mM^{-1}$ $s^{-1}$.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, and all such mixtures fall within the scope of the present disclosure. Additional asymmetric carbon atoms may be present in substituents such as alkyl. All these isomers and mixtures thereof are encompassed within the scope of the present disclosure. The compound comprising asymmetric carbon atoms of the present disclosure can be isolated in optically active-pure or racemic forms. The optically active-pure form can be resolved from the racemic mixture or synthesised by using chiral raw materials or chiral reagents.

Optically active (R)- and (S)-isomers and D and L isomers can be prepared using chiral synthesis or chiral reagents or other conventional techniques. If an enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as amino) or an acidic functional group (such as carboxyl), diastereomeric salts can be formed with an appropriate optically active acid or base, followed by resolution of the diastereomers using conventional methods well known in the art, and subsequent recovery of the pure enantiomers. In addition, separation of enantiomers and diastereomers is generally accomplished by using chromatography, which uses chiral stationary phases, optionally in combination with chemical derivatization methods (e.g., formation of carbamates from amines).

In the chemical structures of the compounds of the present disclosure, a bond "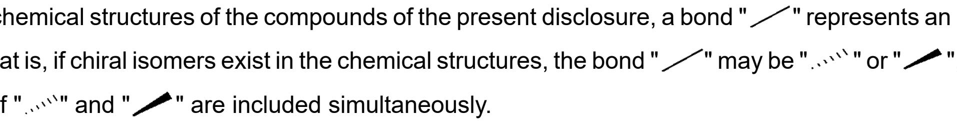" represents an unspecified configuration; that is, if chiral isomers exist in the chemical structures, the bond "" may be "" or "", or both the configurations of "" and "" are included simultaneously.

The compounds and intermediates of the present disclosure may also exist in different tautomeric forms, and all such forms are encompassed within the scope of the present disclosure. The term "tautomer" or "tautomeric form" refers to structural isomers of different energies which are interconvertible via a low energy barrier. For example, proton tautomers (also known as proton transfer tautomers) include interconversion via proton migration, such as keto-enol, imine-enamine and lactam-lactim isomerization. An example of a lactam-lactim equilibrium is present between A and B as shown below.

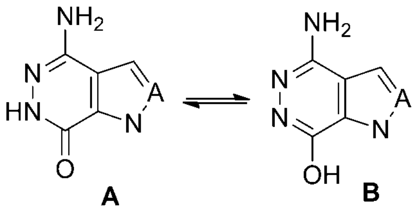

A B

All compounds in the present disclosure may be drawn as form A or form B. All tautomeric forms are included within the scope of the present disclosure. The names of the compounds do not exclude any tautomers.

The present disclosure further includes isotopically labelled compounds of the present disclosure that are identical to those recited herein, but have one or more atoms replaced by an atom having an atomic mass or mass number different from the atomic mass or mass number usually found in nature. Examples of isotopes that can be incorporated into compounds of the present disclosure include isotopes of hydrogen, carbon, nitrogen, oxygen, phosphorus, sulphur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{13}F$, $^{123}I$, $^{125}I$ and $^{36}Cl$, respectively.

Unless otherwise specified, when a position is specifically designated as deuterium (D), it should be understood that the position has a deuterium abundance at least 1000 times greater than the natural abundance of deuterium (which is 0.015%) (i.e., at least 10% deuterium incorporation). The expression "having deuterium at an abundance greater than the natural abundance of deuterium" in the compounds in examples means having deuterium at an abundance at least 1000 times greater, at least 2000 times greater, at least 3000 times greater, at least 4000 times greater, at least 5000 times greater, at least 6000 times greater, or having deuterium at a higher abundance. The present disclosure further includes various deuterated forms of the compound of formula (I). Each available hydrogen atom attached to a carbon atom can be independently replaced with a deuterium atom. Those skilled in the art can synthesise deuterated forms of the compound of formula (I) by referring to relevant literatures. The deuterated forms of the compound of formula (I) can be prepared using commercially available deuterated starting materials, or can be synthesised using deuterated reagents using conventional techniques. The deuterated reagents include, but are not limited to, deuterated borane, solution of trideuterated borane in tetrahydrofuran, deuterated lithium aluminium hydride, deuterated iodoethane and deuterated iodomethane.

"Optionally" or "optional" means that the event or circumstance subsequently described may but need not occur, and the description includes the case where the event or circumstance occurs or does not occur. For example, "$C_{1-6}$ alkyl optionally substituted with halogen or cyano" means that halogen or cyano may but need not be present. The description includes the case where the alkyl is substituted with halogen or cyano and the case where the alkyl is not substituted with halogen or cyano.

Explanation of terms:

The term "pharmaceutical composition" denotes a mixture containing one or more compounds described herein or physiologically or pharmaceutically acceptable salts or prodrug thereof and other chemical components, as well as other components, such as physiologically or pharmaceutically acceptable carriers and excipients. The purpose of pharmaceutical compositions is to facilitate administration to living organisms and facilitate the absorption of active ingredients to exert biological activity.

The term "effective amount" described in the present disclosure includes an amount sufficient to allow or facilitate diagnosis. The effective amount may be the maximum dosage or administration scheme that avoids significant side effects or toxic effects.

The term "pharmaceutically acceptable excipient" includes, but is not limited to, any auxiliary, carrier, glidant, sweetener, diluent, preservative, dye/colourant, flavouring agent, surfactant, wetting agent, dispersant, suspending agent, stabiliser, isotonic agent, solvent, or emulsifier that has been approved by the U.S. Food and Drug Administration as acceptable for use in humans or livestock animals.

The term "alkyl" refers to a linear or branched saturated aliphatic hydrocarbon group comprising 1 to 20 carbon atoms, for example, alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl and various branched-chain isomers thereof. The alkyl may be substituted or unsubstituted, and when it is substituted, the substituent may be substituted at any available point of attachment, and is preferably one or more groups independently selected from halogen, hydroxyl, oxo, nitro, cyano, amino, $C_{1-6}$ alkyl or $C_{1-6}$ alkoxy.

The term "cycloalkyl" or "carbocyclic ring" refers to a saturated or partially unsaturated, monocyclic or polycyclic hydrocarbon substituent. The cycloalkyl ring comprises 3 to 20 carbon atoms, preferably 3 to 7 carbon atoms. Non-limiting examples of monocyclic cycloalkyl groups include cyclopropyl, cyclobutyl, cyclopentyl, cyclopentenyl, cyclohexyl, cyclohexenyl, and cyclohexadienyl. Examples of polycyclic cycloalkyl include spiro ring, fused ring and bridged ring cycloalkyl. Cycloalkyl can be substituted or unsubstituted.

The term "heterocycloalkyl" refers to a saturated or partially unsaturated, monocyclic or polycyclic hydrocarbon substituent, which comprises 3 to 6 ring atoms. Non-limiting examples of "heterocycloalkyl" include:

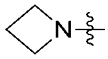,
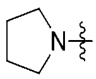

or

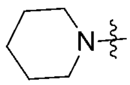.

The heterocycloalkyl may be optionally substituted or unsubstituted. When the heterocycloalkyl is substituted, the substituent is preferably one or more groups independently selected from halogen, hydroxyl, oxo, nitro, cyano, amino, $C_{1-6}$ alkyl or $C_{1-6}$ alkoxy.

The term "heteroaryl" refers to a heteroaromatic system containing 1 to 3 heteroatoms , wherein the heteroatoms are selected from oxygen, sulphur and nitrogen. The heteroaryl is preferably 5-membered heteroaryl. For example, non-limiting examples thereof include:

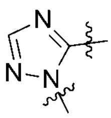 or 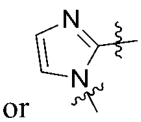.

The heteroaryl may be optionally substituted or unsubstituted. When the heteroaryl is substituted, the substituent is preferably one or more groups independently selected from halogen, hydroxyl, oxo, nitro, cyano, amino, $C_{1-6}$ alkyl or $C_{1-6}$ alkoxy.

The term "alkoxy" refers to -O-(alkyl), wherein the alkyl is as defined above. Non-limiting examples include: methoxy, ethoxy, propoxy and butoxy. The alkoxy may be optionally substituted or unsubstituted. When the alkoxy is substituted, the substituent is preferably one or more groups independently selected from halogen, hydroxyl, oxo, nitro, cyano, amino, $C_{1-6}$ alkyl or $C_{1-6}$ alkoxy.

The term "heterocycloalkoxy" refers to -O-(heterocycloalkyl), wherein the heterocycloalkyl is as defined above. Similarly, the term "cycloalkoxy" refers to - O-(cycloalkyl), wherein the cycloalkyl is as defined above.

The term "monovalent group" refers to a moiety derived from a compound by the formal removal of one monovalent atom or group. The term "-ylene" refers to a moiety derived from a compound by the formal removal of two monovalent atoms or atomic groups or one divalent atom or atomic group.

The term "alkylene" refers to the moiety remaining after removal of two hydrogen atoms from an alkane molecule, including linear and branched -ylene groups comprising 1 to 20 carbon atoms. Non-limiting examples of alkylene containing 1 to 6 carbon atoms include methylene ($-CH_2-$) and ethylene (such as - $CH_2CH_2$- or $-CH(CH_3)-$). Unless

otherwise specified, the alkylene may be substituted or unsubstituted.

The term "substituted group" in the present disclosure includes halogen, hydroxyl, oxo, nitro, cyano, $C_{1-6}$ alkyl, $C_{1-6}$ alkoxy, 3- to 6-membered cycloalkoxy, 3- to 6-membered heterocycloalkoxy, 6-membered aryl or 5- to 6-membered heteroaryl, wherein the $C_{1-6}$ alkyl, $C_{1-6}$ alkoxy, 3- to 6-membered cycloalkoxy, 3-to 6-membered heterocycloalkoxy, 6-membered aryl or 5- to 6-membered heteroaryl are optionally substituted with one or more groups selected from halogen, hydroxyl, cyano, amino, $C_{1-6}$ alkyl or $C_{1-6}$ alkoxy.

The term "hydroxyl" refers to -OH group.

The term "halogen" refers to fluorine, chlorine, bromine or iodine.

The term "cyano" refers to -CN.

The term "amino" refers to $-NH_2$.

The term "nitro" refers to $-NO_2$.

The term "oxo" refers to the =O substituent.

The term "substituted" means that one or more hydrogen atoms in a group, preferably up to 5, more preferably 1 to 3 hydrogen atoms are each independently substituted with a corresponding number of substituents. It goes without saying, the substituents may be only at their chemically possible positions, a person skilled in the art can determine the possible or impossible substitutions (by experiment or theory) without paying too much effort.

**Detailed Description of Embodiments**

The present disclosure will be further described below in conjunction with examples, but these examples are not intended to limit the scope of the present disclosure.

Experimental methods without specific conditions indicated in the examples of the present disclosure are generally conducted under conventional conditions, or conditions recommended by the manufacturers of the starting materials or commercial products. The reagents for which the specific source is not indicated, are conventional reagents that are commercially available.

The structures of the compounds are determined by nuclear magnetic resonance (NMR) or /and mass spectrometry (MS). The NMR shift (δ) is given in the unit of $10^{-6}$ (ppm). NMR is determined with a nuclear magnetic resonance spectrometer Bruker AVANCE-400. The solvents for determination are deuterated dimethyl sulphoxide (DMSO-$d_6$), deuterated chloroform ($CDCl_3$), and deuterated methanol (Methanol-$d_4$), and the internal standard is tetramethylsilane (TMS).

HPLC is determined with Agilent1100 high pressure liquid chromatograph, GAS15B DAD ultraviolet detector and Water Vbridge C18 150*4.6 mm 5 um chromatographic column.

MS is determined with Agilent6120 triple quadrupole mass spectrometer, G1315D DAD detector and Waters Xbridge C18 4.6*50 mm, 5 um chromatographic column. The sample is scanned in a positive/negative ion mode with a mass scan range of 80-1200.

Yantai Huanghai HSGF254 silica gel plate is used as a thin layer chromatography silica plate, and the silica gel plate for the thin layer chromatography (TLC) is of the specification of 0.2 mm ± 0.03 mm, and the specification when separating and purifying a product by thin layer chromatography is 0.4 mm - 0.5 mm. Combiflash Rf150 (TELEDYNE ISCO) or Isolara one (Biotage) is used for the flash column purification system.

For the normal phase chromatography, Yantai Huanghai silica gel of 200-300 mesh or 300-400 mesh silica gel is generally used as the carrier, or Santai Technologies pre-packed ultra-pure normal phase silica gel column (40-63 μm, 60 g, 24 g, 40 g, 120 g or other specifications) is used.

Known starting materials in the present disclosure may be synthesised using or according to methods known in the art, or may be purchased from Shanghai Titan Scientific, ABCR GmbH & Co. KG, Acros Organics, Aldrich Chemical Company, Accela ChemBio Inc., Bide Pharmatech Ltd., and other companies.

If there is no special instruction in the examples, reactions can be carried out under nitrogen atmosphere.

The nitrogen atmosphere means that the reaction bottle is connected to a nitrogen balloon with a volume of about 1 L.

The hydrogen atmosphere means that the reaction bottle is connected to a hydrogen balloon with a volume of about 1 L.

Hydrogen is produced by QPH-1L hydrogen generator from Shanghai Quanpu Scientific Instrument Co., Ltd.

The nitrogen atmosphere or hydrogen atmosphere usually comprises: evacuating, filling with nitrogen or hydrogen, and repeating the operation three times.

Unless otherwise specified in the examples, a solution refers to an aqueous solution.

Unless otherwise indicated, the reaction temperature in the examples refers to room temperature, which is 20°C to 30°C.

The reaction progress in the examples is monitored by thin layer chromatography (TLC). The developing solvent used in the reaction, the eluent system for column chromatography and the developing solvent system for thin layer

chromatography used to purify the compound, and the volume ratio of the solvent are adjusted according to the polarity of the compound, and a small amount of alkaline or acidic reagents such as triethylamine and acetic acid can be added for adjustment.

## Example 1

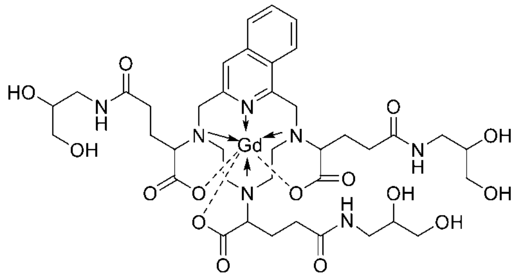

2,2',2''-(3,6,9-triaza-1(1,3)-isoquinoline cyclophane-3,6,9-tris(5-((2,3-dihydroxypropyl)amino)-5-oxopentanoic acid)gadolinium (III) complex (compound 1)

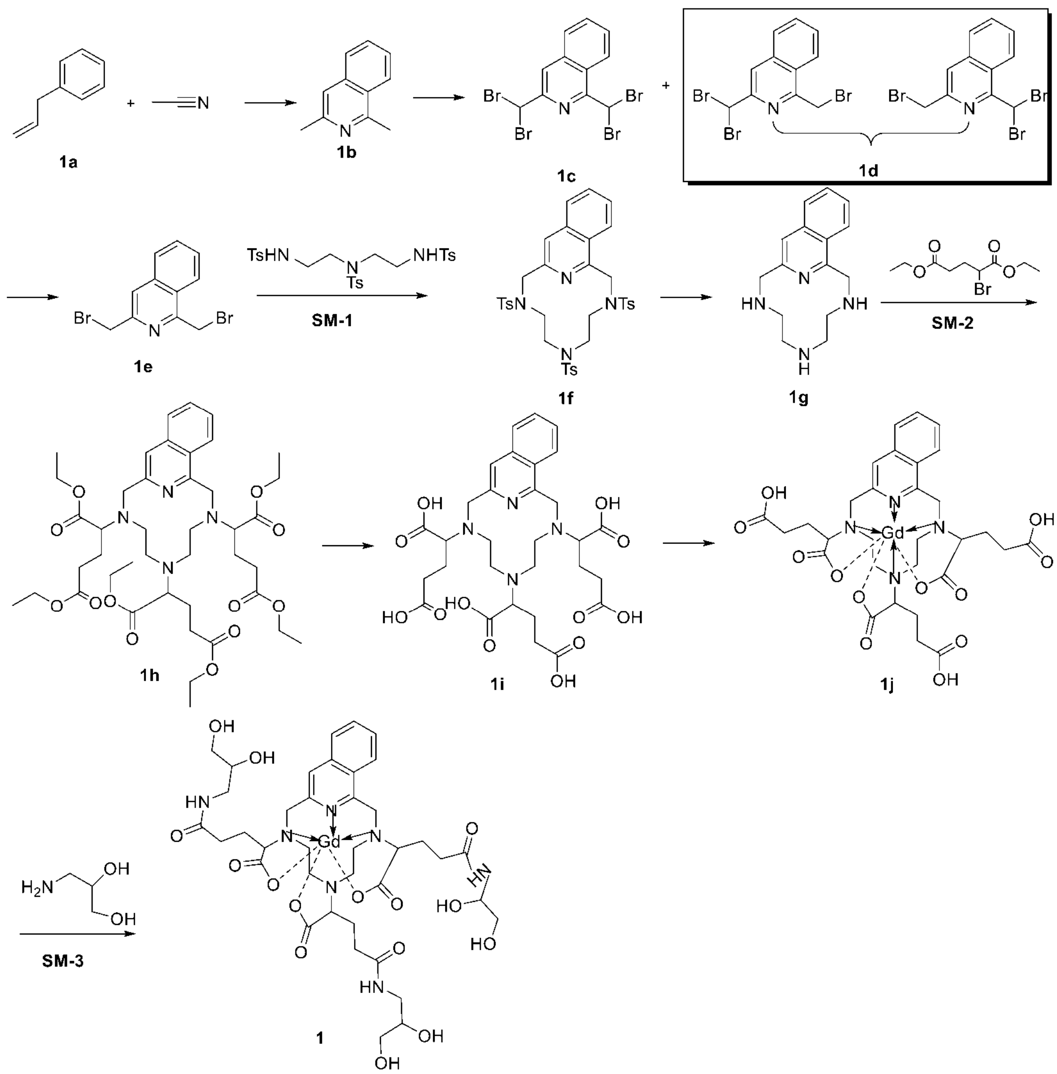

Step 1) Preparation of 1,3-dimethylisoquinoline (compound 1b)

To a 500 mL three-necked flask were added TfOAg (silver trifluoromethanesulphonate, 8.26 g, 32 mmol, 1.0 eq) and acetonitrile (64 mL) at room temperature of 25°C, and then compound 1a (3.8 g, 32 mmol, 1.0 eq) was added to the reaction flask; then the system was cooled to 0°C, and a solution of iodine (8.12 g, 32 mmol, 1 eq) in acetonitrile (128 mL) was added to the system. The reaction mixture was stirred at room temperature until the reaction was essentially complete. The reaction solution was filtered. A solution of KOH (11 g, 167 mmol, 5.2 eq) in methanol (100 mL) was added to the resulting filtrate. After the addition was completed, the mixture was stirred at 40°C. The reaction solution was concentrated under reduced pressure, water was added to the system, and the mixture was extracted with dichloromethane. The organic phase was dried, concentrated, and purified by column chromatography to afford a product (3.16 g, yield: 62.9%).
MS (ESI): m/z 158.1 $[M+1]^+$.
$^1$H NMR (400 MHz, $CDCl_3$) δ 8.06 (d, J = 8.4 Hz, 1H), 7.70 (d, J = 8.2 Hz, 1H), 7.61 (t, J = 7.5 Hz, 1H), 7.50 (t, J = 7.6 Hz, 1H), 7.33 (s, 1H), 2.94 (s, 3H), 2.65 (s, 3H).

Step 2) Preparation of a mixture of 1-(bromomethyl)-3-(dibromomethyl)isoquinoline and 3-(bromomethyl)-1-(dibromomethyl) isoquinoline (compound 1d)

At room temperature of 25°C, to a 500 mL single-necked flask was added compound 1b as a solid (5.2 g, 33.08 mmol, 1.0 eq), followed by $CCl_4$ (220 mL), and the mixture was stirred for dissolution. To the reaction solution were added NBS (30 g, 169.0 mmol, 5 eq) and BPO (480 mg, 1.32 mmol, 0.04 eq), and the mixture was refluxed at 95°C until the reaction was essentially complete. The solution was cooled to 30°C, filtered, washed with saturated sodium chloride aqueous solution, dried, and concentrated to afford 14.4 g of the product (compound 1d); the crude product was directly used in the next step.
MS (ESI): m/z 391.9 $[M+H]^+$.

Step 3) Preparation of a mixture of 1,3-bisbromomethylisoquinoline (compound 1e)

To a reaction flask was added a mixture of compound 1d (crude, 14.4 g, 33.07 mmol, 1.0 eq), and the mixture was dissolved in dry tetrahydrofuran (120 mL). Under nitrogen protection, the system was cooled to approximately -5°C; DIPEA (17.1 g, 132.28 mmol, 4.0 eq) and diethyl phosphite (18.27 g, 17.1 mL) were added to the solution, and the reaction was maintained at approximately 0°C until substantially complete. The reaction solution was poured into an ice-water mixture, and extracted with ethyl acetate. The organic phases were combined, dried, and concentrated to afford 5.2 g of the product (yield: 50%).
MS (ESI): m/z 313.9 $[M+H]^+$.
$^1$H NMR (400 MHz, $CDCl_3$) δ 8.24 (d, J = 8.1 Hz, 1H), 7.85 (d, J = 7.9 Hz, 1H), 7.78 - 7.66 (m, 3H), 5.02 (s, 2H), 4.71 (s, 2H).

Step 4) Preparation of 3,6,9-mesitylenesulphonyl-3,6,9-triaza-1(1,3)-isoquinoline cyclophane (compound 1f)

To a reaction flask were sequentially added SM-1 (9.83 g, 17.378 mmol, 1 eq), potassium carbonate (10.09 g, 72.99 mmol, 4.2 eq) and DMF (240 mL) at room temperature, and the mixture was stirred until a clear solution was obtained; under nitrogen protection, the resulting solution was heated to 100°C. Compound 1e (6.35 g, 20.15 mmol, 1.16 eq) was dissolved in DMF (120 mL), and the solution was added to the above reaction system. The reaction mixture was stirred until the reaction was essentially complete. The reaction solution was cooled to 30°C, then poured into water, filtered under reduced pressure, washed with a small amount of water, and dried to obtain the product (11.4 g, yield: 91%).
MS (ESI): m/z 719.2 $[M+H]^+$.
$^1$H NMR (400 MHz, $CDCl_3$) δ 8.55 (d, J = 8.2 Hz, 1H), 7.84 (dd, J = 15.8, 6.2 Hz, 4H), 7.75 (dd, J = 14.7, 8.3 Hz, 4H), 7.57 (d, J = 8.2 Hz, 2H), 7.37 (dd, J = 13.7, 8.0 Hz, 4H), 7.21 (d, J = 8.0 Hz, 2H), 5.28 - 4.57 (m, 4H), 4.45 (s, 2H), 3.75 (t, J = 6.3 Hz, 2H), 3.39 (dd, J = 24.6, 17.3 Hz, 4H), 2.47 (d, J = 6.7 Hz, 6H), 2.36 (s, 3H).

Step 5) Preparation of 3,6,9-triaza-1(1,3)- isoquinoline cyclophane (compound 1g)

In a 500 mL reaction tube, compound 1f (13.25 g, 18.4 mmol, 1.0 eq) was dispersed in 116 mL of concentrated sulphuric acid. The reaction system was subjected to nitrogen replacement three times, then heated to 105°C and maintained until the reaction was essentially complete. The reaction solution was cooled to room temperature, poured into ice water, and the pH of the mixture was adjusted to approximately 12 with 40% NaOH solution. Then the solution was extracted with DCM/MeOH = 20/1. The extracts were combined, washed with saturated sodium chloride aqueous solution, dried, filtered, and concentrated to afford the target compound 1g (4.57 g, yield: 97%).

MS (ESI): m/z 257.2 $[M+H]^+$.

$^1H$ NMR (400 MHz, $CDCl_3$) δ 8.05 (d, J = 8.4 Hz, 1H), 7.81 - 7.54 (m, 3H), 7.39 (s, 1H), 4.51 (s, 2H), 4.15 (s, 2H), 2.88 (d, J = 4.6 Hz, 2H), 2.77 (t, J = 5.3 Hz, 2H), 2.65 - 2.57 (m, 2H), 2.55-2.51 (m, 2H).

Step 6) Preparation of hexaethyl 2,2',2"-(3,6,9-triaza-1(1,3)-isoquinoline cyclophane-3,6,9-triyl)triglutarate (compound 1h)

In a 250 mL single-necked flask, compound 1g (4.82 g, 18.81 mmol, 1.0 eq) was dissolved in $CH_3CN$ (193 mL). $K_2CO_3$ (11.7 g, 84.64 mmol, 4.5 eq) was added, and the mixture was stirred at room temperature. SM-2 (20.6 g, 77.1 mmol, 4.1 eq) was dissolved in $CH_3CN$ (193 mL), which was then added dropwise to the above solution. After the addition was completed, the reaction system was warmed to 66°C and stirred until the reaction was essentially complete. The mixture was filtered, washed with acetonitrile, and concentrated under reduced pressure to obtain a crude product. The crude product was purified to afford compound 1h (7.9 g, yield: 51.5%).

MS (ESI): m/z 815.4 $[M+H]^+$.

$^1H$ NMR (400 MHz, $CDCl_3$) δ 8.13 (dd, J = 8.6, 3.0 Hz, 1H), 7.81 (d, J = 8.2 Hz, 1H), 7.65 (t, J = 7.6 Hz, 1H), 7.59-7.55 (m, 2H), 4.31 - 3.97 (m, 15H), 3.93 - 3.84 (m, 1H), 3.75 (td, J = 4.1, 2.0 Hz, 3H), 2.88 - 2.73 (m, 3H), 2.52 - 2.37 (m, 4H), 2.29-2.25 (m, 3H), 2.20 - 2.07 (m, 3H), 2.06 - 1.95 (m, 2H), 1.88-1.84 (m, 5H), 1.38-1.34 (m, 6H), 1.31 - 1.18 (m, 12H).

Step 7) Preparation of 2,2',2"-(3,6,9-triaza-1(1,3)-isoquinoline cyclophane-3,6,9-triyl)triglutaric acid (compound 1i)

In a 250 mL single-necked flask, compound 1h (3.83 g, 4.7 mmol, 1.0 eq) was added and dissolved in THF (50 mL). 1 M LiOH solution (56.4 mL) was added dropwise to the mixture at room temperature of 25°C. After the addition was completed, the reaction system was stirred at room temperature for 16 h, and then concentrated under reduced pressure. The pH of the residue was adjusted to 2-3 with concentrated hydrochloric acid in an ice bath, and the mixture was purified directly by macroporous resin XAD 1600 column chromatography to afford the product compound 1i (2.85 g, yield: 93.8%).

MS (ESI): m/z 647.3 $[M+H]^+$.

$^1H$ NMR (400 MHz, $D_2O$) δ 8.14 - 8.00 (m, 1H), 7.99 - 7.78 (m, 3H), 7.77-7.72 (m, 1H), 5.43 - 4.79 (m, 2H), 4.69 (s, 7H), 4.12 - 3.77 (m, 2H), 3.78 - 3.37 (m, 4H), 3.25 (d, J = 31.7 Hz, 1H), 2.81 - 2.39 (m, 5H), 2.38 - 1.79 (m, 6H).

Step 8) Preparation of 2,2',2"-(3,6,9-triaza-1(1,3)-isoquinoline cyclophane-3,6,9-triyl)triglutaric acid gadolinium (III) complex (compound 1j)

To a 50 mL single-necked flask were added compound 1i (1.57 g, 2.43 mmol, 1.0 eq) and deionized $H_2O$ (35 mL). The pH of the mixture was adjusted to 10 with 2 N NaOH solution. A solution of $GdCl_3 \cdot 6H_2O$ (0.994 g, 2.67 mmol, 1.0 eq) in 35 mL of deionized water was slowly added to the reaction system. The pH of the resulting solution was adjusted to approximately 6-7 with 1 N HCl. The mixture was stirred at room temperature (23°C) for about 2 h. The reaction solution was lyophilised, and the residue was purified by preparative liquid chromatography to afford compound 1j (1.05 g, yield: 54%).

MS (ESI): m/z 802.2 $[M+H]^+$.

Step 9) Preparation of compound 1

In a 15 mL single-necked flask, starting material SM-3 (33 mg, 0.364 mmol, 4.0 eq) was added and dissolved in $H_2O$ (1 mL). The pH of the solution was adjusted to 6-7 with 1 N HCl. Compound 1j (73 mg, 0.091 mmol, 1.0 eq), HOBT (6 mg, 0.046 mmol, 0.5 eq) and EDCI (88 mg, 0.455 mmol, 5.0 eq) were added to the system, and then the reaction was stirred at room temperature (20°C) for 16 h. The mixture was purified by preparative liquid chromatography, and then lyophilised to obtain the product (74 mg, purity: 95.67%).

MS (ESI): m/z 1021.3 $[M+H]^+$.

**Example 2**

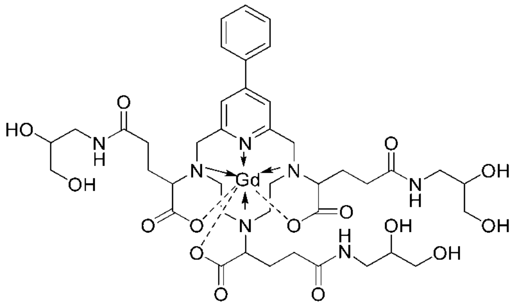

2,2',2'-(1[4]-phenyl-3,6,9-triaza-1(2,6)-pyridine cyclodecane-3,6,9-triyl)tris(5-((2,3-dihydroxypropyl)amino)-5-oxopentanedioic acid)gadolinium (III) complex (compound 2)

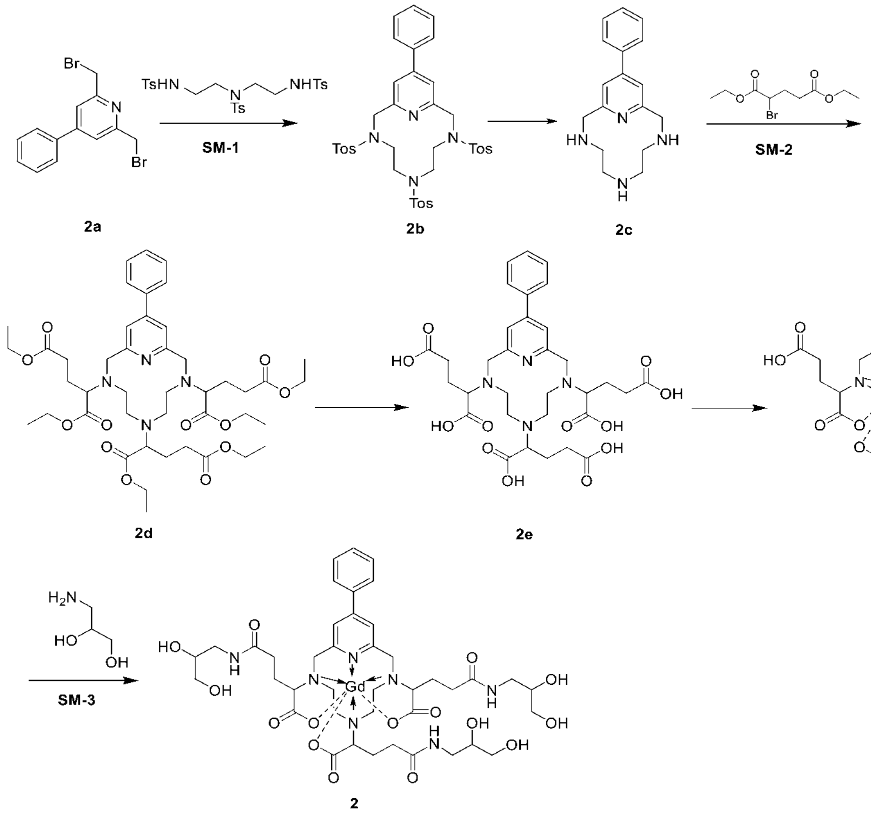


Step 1) Preparation of 1[4]-phenyl-3,6,9-tris(p-toluenesulphonyl)-3,6,9-triaza-1(2,6)-pyridine cyclodecane (compound 2b)

SM-1 (5.23 g, 9.2 mmol, 1.05 eq) was placed in a 500 mL single-necked flask, and DMF (157 mL) was added. The mixture was stirred for dissolution, and heated to 100°C. Compound 2a (3.0 g, 8.8 mmol, 1.0 eq, synthesised with reference to Bioorganic and Medicinal Chemistry, 2020, vol. 28, # 24, no. 115816) was dissolved in 60 mL of DMF, and the resulting solution was slowly added dropwise to the above solution maintained at 100°C. After the dropwise addition was completed, the reaction system was cooled to 90°C and stirred until the reaction was essentially complete. The reaction system was cooled to room temperature, poured into ice water, and extracted with ethyl acetate, and the organic phases were combined. The combined organic phases were washed with water, then with saturated NaCl aqueous solution, and dried. The mixture was filtered and concentrated to afford crude compound 2b (5.8 g, yield: 88.5%).

MS (ESI): m/z 745.2 $[M+1]^+$.

$^1H$ NMR (400 MHz, $CDCl_3$) $\delta$ 7.74 (d, *J* = 8.0 Hz,4H), 7.68 - 7.59 (m, 6H), 7.53 - 7.44 (m, 3H), 7.35 (d, *J* = 8.0 Hz, 4H), 7.28 (m, 2H), 4.35 (s, 4H), 3.36 (t, J = 7.4 Hz, 4H), 2.90 - 2.71 (m, 4H), 2.45 (s, 6H), 2.39 (s, 3H).

Step 2) Preparation of $1^4$-phenyl-3,6,9-triaza-1(2,6)-pyridine cyclodecane (compound 2c)

Compound 2b (2.0 g, 2.7 mmol, 1.0 eq) was placed in a 100 mL flask, and 20 mL of concentrated sulphuric acid was added. The reaction system was subjected to nitrogen replacement three times, and then heated to 100°C. The reaction mixture was stirred until the reaction was essentially complete. The reaction solution was cooled to room temperature, and poured into 100 mL of ice water. While maintaining the temperature at 0°C, the pH of the mixture was adjusted to 12 with 40% aqueous NaOH solution. The mixture was extracted with dichloromethane. The extracts were combined, and dried over anhydrous sodium sulphate. The mixture was filtered and concentrated to afford the product (390 mg, yield: 51.5%).
MS (ESI): m/z 283.2 $[M+1]^+$.

Step 3) Preparation of hexaethyl 2,2',2"-($1^4$-phenyl-3,6,9-triaza-1(2,6)-pyridine cyclodecan-3,6,9-triyl)tris-glutamate (compound 2d)

Compound 2c (390 mg, 1.4 mmol, 1.0 eq) was placed in a 100 mL single-necked flask, and acetonitrile (18 mL) was added. The mixture was stirred for dissolution. $K_2CO_3$ (858 mg, 6.2 mmol, 4.5 eq) was then added to the system. SM-2 (1.33g, 5.0 mmol, 3.6 eq) was added dropwise to the above reaction solution. After the addition was completed, the reaction system was warmed to 66°C and stirred until the reaction was essentially complete. The reaction solution was concentrated. The resulting crude product was diluted with water, and extracted with dichloromethane. The organic phases were combined, washed with saturated NaCl solution, dried, filtered and concentrated to obtain a crude product. The crude product was purified by normal-phase column chromatography to afford compound 2d (695 mg, yield: 59.9%).
MS (ESI): m/z 841.4 $[M+1]^+$.

Step 4) Preparation of 2,2',2"-($1^4$-phenyl-3,6,9-triaza-1(2,6)-pyridine cyclodecane-3,6,9-triyl)tris-glutamic acid (compound 2e)

To a 50 mL single-necked flask were added compound 2d (620 mg, 0.74 mmol, 1.0 eq) and EtOH (9 mL), and the mixture was stirred for dissolution, and then cooled to 0°C in an ice bath. 5 M NaOH (2.4 mL, 11.8 mmol, 16.0 eq) was added dropwise to the reaction solution. After the addition was completed, the reaction system was warmed to 80°C and stirred until the reaction was essentially complete. The mixture was concentrated under reduced pressure to remove ethanol. The crude product was cooled to 0°C, and the pH was adjusted to 3. The crude product was purified by macroporous resin XAD 1600 column chromatography to obtain compound 2e (345 mg, yield: 69.6%).
MS (ESI): m/z 673.3 $[M+1]^+$.

Step 5) Preparation of 2,2',2'-($1^4$-phenyl-3,6,9-triaza-1(2,6)-pyridine cyclodecane-3,6,9-triyl)tris(4-carboxybutanoic acid)gadolinium (III) complex (compound 2f)

Compound 2e (280 mg, 0.42 mmol, 1.0 eq) was placed in a 50 mL single-necked flask, and deionized water (8 mL) was added. The mixture was stirred for dissolution. The pH of the reaction solution was adjusted to 7 with 1 N NaOH solution. $GdCl_3 \cdot 6H_2O$ (156 mg, 0.42 mmol, 1.0 eq) was added to the system. After the addition was completed, the pH of the reaction solution was adjusted to 7 again with 1 N NaOH solution, and the mixture was stirred at room temperature for 16 h. The resulting mixture was purified by macroporous resin XAD 1600 column chromatography to afford compound 2f (65 mg, yield: 18.9%).
MS (ESI): m/z 828.2 $[M+1]^+$.

Step 6) Preparation of compound 2

SM-3 (28.8 mg, 0.32 mmol, 4.0 eq) was placed in a 25 mL single-necked flask, and deionized water (0.5 mL) was added. The mixture was stirred for dissolution. The pH of the resulting solution was adjusted to 7 with 1 N HCl. Compound 2f (65 mg, 0.08 mmol, 1.0 eq), HOBT (5.4 mg, 0.04 mmol, 0.5 eq) and EDCI (75.7 mg, 0.4 mmol, 5.0 eq) were added to the reaction system, and the mixture was stirred at room temperature for 3 days. The crude product was purified by preparative liquid chromatography to afford compound 2 (64 mg, yield: 77.9%).
MS (ESI): m/z 1047.3 $[M+1]^+$.

## Example 3

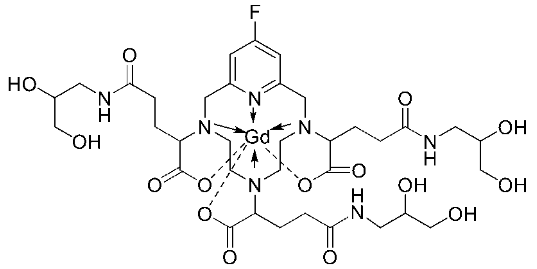

2,2',2'-($1^4$-fluoro-3,6,9-triaza-1(2,6)-pyridine cyclodecane-3,6,9-triyl)tris(5-((2,3-dihydroxypropyl)amino)-5-oxopentanedioic acid)gadolinium (III) complex (compound 3)

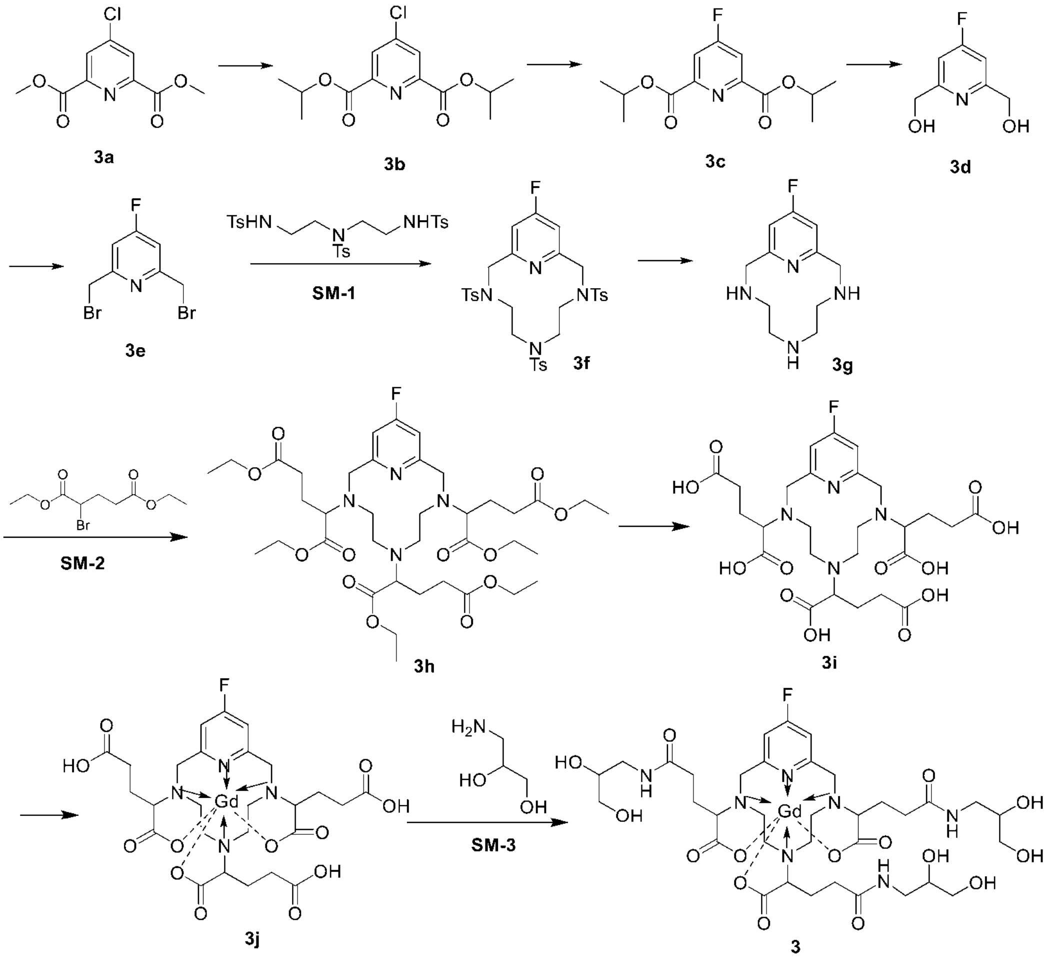


3a 3b 3c 3d 3e 3f 3g 3h 3i 3j 3

Step 1) Preparation of diisopropyl 4-chloropyridine-2,6-dicarboxylate (compound 3b)

To a 500 mL single-necked flask were added compound 3a (10 g, 43.5 mmol, 1.0 eq), isopropanol (200 mL) and concentrated sulphuric acid (5 mL) at room temperature of 25°C under nitrogen protection. The reaction system was heated to 80°C, and stirred until the reaction was essentially complete. The mixture was concentrated under reduced pressure to afford a light yellow solid. Water was added, and the resulting mixture was extracted with ethyl acetate. The organic layers were combined, washed with saturated sodium bicarbonate solution, then with saturated sodium chloride solution, dried, suction-filtered, and concentrated. The crude product was purified by column chromatography to afford compound 3b (7.87 g, yield: 63.3%).

MS: 286.1 $[M+H]^+$.

Step 2) Preparation of diisopropyl 4-fluoropyridine-2,6-dicarboxylate (compound 3c)

To a 1 L three-necked flask were added compound 3b (23.6 g, 82.6 mmol, 1.0 eq), CsF (37.66 g, 247.8 mmol, 3 eq) and 4A molecular sieve (8 g). Under nitrogen protection, the reaction system was heated to 105°C, and stirred for 2.5 hours. The reaction solution was cooled to 25°C, and filtered under reduced pressure. The filter cake was washed with ethyl acetate. The filtrate was collected, diluted with ethyl acetate, washed with water, then with saturated sodium chloride solution, dried, suction-filtered, and concentrated under reduced pressure to afford compound 3c (21 g, yield: 94%).
MS: 270.1 $[M+H]^+$.
$^1H$ NMR (400 MHz, $CDCl_3$) δ 7.95 (d, J = 8.3 Hz, 2H), 5.32 (p, J = 6.2 Hz, 2H), 1.44 (d, J = 6.2 Hz, 12H).
$^{19}F$ NMR (376 MHz, $CDCl_3$) δ -98.84 (s).

Step 3) Preparation of (4-fluoropyridine-2,6-diyl)dimethanol (compound 3d)

To a 1 L single-necked flask were added compound 3c (10 g, 37.8 mmol, 1.0 eq) and isopropanol (350 mL), and then sodium borohydride (6.6 g, 173.7 mmol, 4.6 eq) was added slowly in batches at room temperature of 25°C. After the addition was completed, the reaction system was warmed to 50°C, and stirred for 5 hours under nitrogen protection. The reaction solution was cooled to 0°C, and acetone was added to quench the reaction. After stirring for 10 minutes, saturated sodium bicarbonate solution was slowly added at 0°C to further quench the reaction. The mixture was diluted with water, and extracted with ethyl acetate. The organic layers were combined, washed with saturated sodium chloride solution, dried, suction-filtered, and concentrated to afford compound 3d (5 g, yield: 84.2%).
MS: 158.1 $[M+H]^+$.
$^1H$ NMR (400 MHz, DMSO-$d_6$) δ 7.13 (d, J = 10.0 Hz, 2H), 5.59 (brs, 2H), 4.53 (s, 4H).
$^{19}F$ NMR (376 MHz, DMSO-$d_6$) δ -103.53.

Step 4) Preparation of 2,6-bis(bromomethyl)-4-fluoropyridine (compound 3e)

In a 500 mL single-necked flask, compound 3d (5 g, 31.8 mmol, 1 eq) was dispersed in 120 mL of chloroform. Under nitrogen protection, the mixture was cooled to 0°C, and $PBr_3$ (129 g, 44.9 mL, 477.3 mmol, 15 eq) was added dropwise. After the dropwise addition was completed, the reaction system was warmed to 60°C and stirred until the reaction was essentially complete. The reaction solution was slowly poured into saturated sodium bicarbonate solution, and the pH of the mixture was adjusted to 8 with $NaHCO_3$ solid. The mixture was extracted with dichloromethane. The extracts were combined, washed with saturated NaCl solution, dried, filtered, and concentrated. The crude product was purified by normal-phase column chromatography to afford compound 3e (2.52 g, yield: 28%).
MS: 281.9 $[M+H]^+$.
$^1H$ NMR (400 MHz, $CDCl_3$) δ 7.13 (d, J = 8.8 Hz, 2H), 4.51 (s, 4H).
$^{19}F$ NMR (376 MHz, $CDCl_3$) δ -100.47.

Step 5) Preparation of $1^4$-fluoro-3,6,9-tris(p-toluenesulphonyl)-3,6,9-triaza-1(2,6)-pyridine cyclophane (compound 3f)

To a 500 mL single-necked eggplant-shaped flask were sequentially added SM-1 (6.39 g, 11.29 mmol, 1 eq), potassium carbonate (6.55 g, 47.42 mmol, 4.2 eq) and DMF (160 mL) at room temperature, the mixture was stirred until a clear solution was obtained. With stirring at room temperature, the reaction system was subjected to nitrogen replacement three times, and heated to 100°C. Compound 3e (3.45 g, 12.2 mmol, 1 eq) was dissolved in DMF (61 mL), and the solution was slowly added to the above reaction system. After the addition was completed, the reaction mixture was stirred until the reaction was essentially complete. The reaction solution was cooled to 30°C, poured into water with stirring, and filtered. The filter cake was washed with water, and dried overnight to obtain compound 3f (6.1 g, yield: 79%).
Ms (ESI): m/z 687.2 $[M+H]^+$.
$^1H$ NMR (400 MHz, $CDCl_3$) δ 7.69 (dd, J = 12.4, 8.2 Hz, 6H), 7.32 (dd, J = 21.0, 8.1 Hz, 6H), 7.14 (d, J = 8.5 Hz, 2H), 4.28 (s, 4H), 3.33 (t, J = 7.2 Hz, 4H), 2.89 (s, 4H), 2.45 (s, 6H), 2.42 (s, 3H).
$^{19}F$ NMR (376 MHz, $CDCl_3$) δ -98.30.

Step 6) Preparation of $1^4$-fluoro-3,6,9-tris(p-toluenesulphonyl)-3,6,9-triaza-1(2,6)-pyridine cyclophane (compound 3g)

In a 100 mL reaction tube, compound 3f (3 g, 4.37 mmol, 1.0 eq) was dispersed in 30 mL of concentrated sulphuric acid. The reaction system was subjected to nitrogen replacement three times, then heated to 105°C and reacted for 3 h. The reaction solution was cooled to room temperature, and poured into ice water, and the pH of the mixture was slowly adjusted to approximately 12 with 40% NaOH solution. Then the mixture was extracted with DCM/MeOH = 20/1. The extracts were combined, and washed with saturated sodium chloride aqueous solution. The organic phase was dried, and

concentrated to afford compound 3g (1.08 g, yield: 55.1%).
MS (ESI): m/z 225.2 $[M+H]^+$.

Step 7) Preparation of hexaethyl 2,2',2"-($1^4$-fluoro-3,6,9-triaza-1(2,6)-pyridine cyclodecane-3,6,9-triyl)tris-glutamate (compound 3h)

Compound 3g (138 mg, 0.62 mmol, 1.0 eq) was placed in a 25 mL single-necked flask, and acetonitrile (6 mL) was added. The mixture was stirred for dissolution. $K_2CO_3$ (386 mg, 2.8 mmol, 4.5 eq) was then added to the system. SM-2 (596 mg, 2.2 mmol, 3.6 eq) was added dropwise to the above reaction solution. After the addition was completed, the reaction system was warmed to 66°C and stirred until the reaction was essentially complete. The reaction solution was concentrated. The residue was diluted with water, and extracted with dichloromethane. The organic phases were combined, washed with saturated NaCl solution, dried over anhydrous sodium sulphate, filtered and concentrated to obtain a crude product. The crude product was purified by column chromatography to afford compound 3h (160 mg, yield: 33.2%).
MS (ESI): m/z 783.4 $[M+1]^+$

Step 8) Preparation of 2,2',2"-($1^4$-fluoro-3,6,9-triaza-1(2,6)-pyridine cyclodecane-3,6,9-triyl)tris-glutamic acid (compound 3i)

To a 50 mL single-necked flask were added compound 3h (320 mg, 0.41 mmol, 1.0 eq) and THF (10 mL), and the mixture was stirred for dissolution, and then cooled to 0°C. 1M aqueous solution of LiOH (4.9 mL, 4.9 mmol, 12.0 eq) was added dropwise to the reaction mixture. The reaction mixture was stirred at room temperature until the reaction was essentially complete. The pH of the resulting solution was adjusted to 6 with 1 M hydrochloric acid, and the mixture was concentrated under reduced pressure. The residue was purified by macroporous resin XAD 1600 column chromatography to obtain compound 3i (115 mg, yield: 45.8%).
MS (ESI): m/z 615.2 $[M+1]^+$.

Step 9) Preparation of 2,2',2'-($1^4$-fluoro-3,6,9-triaza-1(2,6)-pyridine cyclodecane-3,6,9-triyl)tris(4-carboxybutanoic acid)gadolinium (III) complex (compound 3j)

Compound 3i (89 mg, 0.15 mmol, 1.0 eq) was placed in a 25 mL single-necked flask, and deionized water (2 mL) was added. The mixture was stirred for dissolution. The pH of the reaction solution was adjusted to 7 with 1 N NaOH solution. $GdCl_3 \cdot 6H_2O$ (54 mg, 0.15 mmol, 1.0 eq) was added to the system. After the addition was completed, the pH of the reaction solution was adjusted to 7 again with 1 N NaOH solution, and the mixture was stirred at room temperature for 16 h. The resulting mixture was purified by macroporous resin XAD 1600 column chromatography to afford the product compound 3j (75 mg, yield: 67.4%).
MS (ESI): m/z 770.1 $[M+1]^+$.

Step 10) Preparation of compound 3

SM-3 (36 mg, 0.39 mmol, 4.0 eq) was placed in a 25 mL single-necked flask, and deionized water (0.5 mL) was added. The mixture was stirred for dissolution. The pH of the resulting solution was adjusted to 7 with 1 N HCl. Compound 3j (75 mg, 0.1 mmol, 1.0 eq), HOBT (6.6 mg, 0.05 mmol, 0.5 eq) and EDCI (94 mg, 0.5 mmol, 5.0 eq) were added to the reaction system separately, and the mixture was stirred at room temperature for 3 days. The resulting mixture was purified by preparative liquid chromatography to afford the product compound 3 (65 mg, yield: 66.7%).
MS (ESI): m/z 989.3 $[M+1]^+$.

Example 4

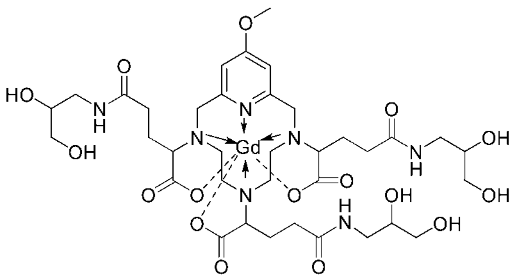

2,2',2'-(1⁴-methoxy-3,6,9-triaza-1(2,6)-pyridine cyclodecane-3,6,9-triyl)tris(5-((2,3-dihydroxypropyl)amino)-5-oxopentanedioic acid)gadolinium (III) complex (compound 4)

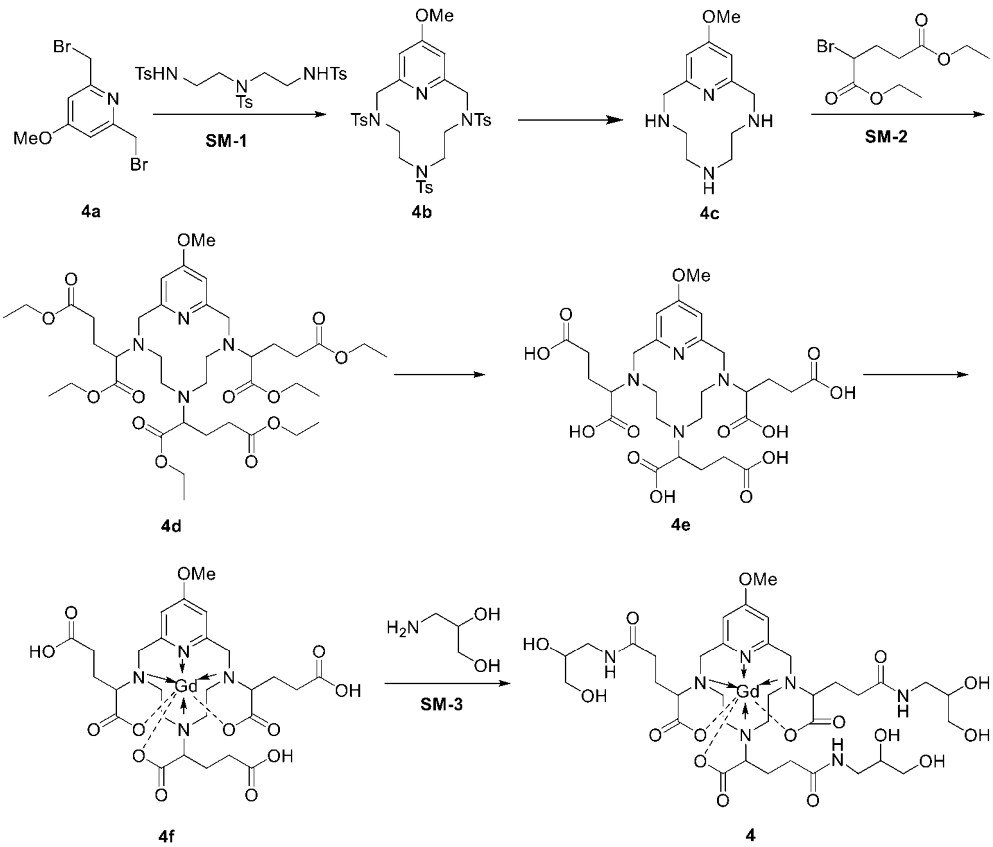


Step 1) Preparation of $1^4$-methoxy-3,6,9-tris(p-toluenesulphonyl)-3,6,9-triaza-1(2,6)-pyridine cyclodecane (compound 4b)

SM-1 (16.86 g, 29.8 mmol, 1.1 eq) was placed in a 1 L single-necked flask, and DMF (506 mL) was added. The mixture was stirred for dissolution, and heated to 100°C. Compound 4a (8.0 g, 27.1 mmol, 1.0 eq, synthesised with reference to Bioorganic and Medicinal Chemistry, 2020, vol. 28, # 24, no. 115816) was dissolved in 160 mL of DMF, and the resulting solution was slowly added dropwise to the above solution maintained at 100°C. After the dropwise addition was completed, the reaction mixture was stirred until the reaction was essentially complete. The reaction system was cooled to room temperature, poured into ice water, and extracted with ethyl acetate, and the organic phases were combined. The combined organic phases were washed with water, then with saturated NaCl aqueous solution, dried over anhydrous sodium sulphate, filtered, and concentrated to afford compound 4b (16.8 g, yield: 88.7%).

MS (ESI): m/z 699.2 $[M+1]^+$.

$^1$H-NMR (400 MHz, $CDCl_3$) $\delta$ 7.71 (d, *J* = 8.4 Hz, 4H), 7.66 (d, *J* = 8.0 Hz, 2H), 7.34 (d, *J* = 8.0 Hz, 4H), 7.28 (d, *J* = 8.0 Hz, 2H), 6.91 (s, 2H), 4.22 (br., 4H), 3.85 (s, 3H), 3.32 (t, *J* = 7.2 Hz, 4H), 2.79 (br., 4H), 2.45 (s, 6H), 2.42 (s, 3H).

Step 2) Preparation of 1[4]-methoxy-3,6,9-triaza-1(2,6)-pyridine cyclodecane (compound 4c)

Compound 4b (8.0 g, 11.45 mmol, 1.0 eq) was placed in a 250 mL flask, and 80 mL of concentrated sulphuric acid was added. The reaction system was subjected to nitrogen replacement three times, then heated to 100°C, and stirred until the reaction was essentially complete. The reaction solution was cooled to room temperature, and poured into ice water. While maintaining the temperature at 0°C, the pH of the mixture was adjusted to 12 with 40% aqueous NaOH solution. The mixture was extracted with dichloromethane, dried over anhydrous sodium sulphate, and concentrated to afford compound 4c (2.5 g, yield: 92.6%).
MS (ESI): m/z 237.2 $[M+1]^+$.

Step 3) Preparation of hexaethyl 2,2',2"-(1[4]-methoxy-3,6,9-triaza-1(2,6)-pyridine cyclodecane-3,6,9-triyl)tris-glutamate (compound 4d)

Compound 4c (2.32 g, 9.82 mmol, 1.0 eq) was placed in a 250 mL single-necked flask, and acetonitrile (50 mL) was added. The mixture was stirred for dissolution, and then $K_2CO_3$ (6.79 g, 49.1 mmol, 5.0 eq) was added to the system. SM-2 (10.49 g, 39.28 mmol, 4.0 eq) was dissolved in acetonitrile (10 mL), which was then added dropwise to the above reaction solution. The reaction system was warmed to 66°C and stirred until the reaction was essentially complete. The reaction solution was concentrated. The crude product was diluted with water, and extracted with dichloromethane. The organic phases were combined, washed with saturated NaCl solution, dried, and concentrated. The resulting mixture was purified by normal-phase column chromatography to afford the product compound 4d (3.7 g, yield: 47.7%).
MS (ESI): m/z 795.4 $[M+1]^+$.
$^1H$ NMR (400 MHz, $CDCl_3$) δ 6.70 (s, 2H), 4.23 - 4.01 (m, 14H), 3.98-3.76 (m, 5H), 3.69 (s, 2H), 3.34 (s, 2H), 3.11 - 2.74 (m, 5H), 2.55 - 2.21 (m, 9H), 2.12 - 1.91 (m, 5H), 1.36 - 1.18 (m, 18H).

Step 4) Preparation of 2,2',2"-(1[4]-methoxy-3,6,9-triaza-1(2,6)-pyridine cyclodecane-3,6,9-triyl)tris-glutamic acid (compound 4e)

To a 50 mL single-necked flask were added compound 4d (3.55 g, 4.47 mmol, 1.0 eq) and EtOH (12.5 mL), and the mixture was stirred for dissolution, and then cooled to 0°C in an ice bath. 5 M NaOH (14.3 mL,71.52 mmol,16.0 eq) was added dropwise to the reaction solution. After the addition was completed, the reaction system was warmed to 80°C and stirred until the reaction was essentially complete. The reaction solution was concentrated. The crude product was cooled to 0°C, and the pH was adjusted to 3. The crude product was purified by macroporous resin XAD 1600 column chromatography to obtain the product compound 4e (2.48 g, yield: 88.6%).
MS (ESI): m/z 627.2 $[M+1]^+$.
$^1H$ NMR (400 MHz, $D_2O$) δ 7.19 - 6.91 (m, 2H), 4.57 - 4.16 (m, 4H), 3.97 - 3.86 (m, 3H), 3.84 - 3.82 (m, 3H), 3.53 - 2.94 (m, 8H), 2.69 - 2.34 (m, 6H), 2.26 - 1.82 (m, 6H).

Step 5) Preparation of 2,2',2'-(1[4]-methoxy-3,6,9-triaza-1(2,6)-pyridine cyclodecane-3,6,9-triyl)tris(4-carboxybutanoic acid)gadolinium (III) complex (compound 4f)

Compound 4e (2.48 g, 3.98 mmol, 1.0 eq) was placed in a 50 mL single-necked flask, and deionized water (24 mL) was added. The mixture was stirred for dissolution. The pH of the reaction solution was adjusted to 7 with 2 N NaOH solution. $GdCl_3 \cdot 6H_2O$ (1.48 g, 3.98 mmol, 1.0 eq) was added to the system. After the addition was completed, the pH of the reaction solution was adjusted to 7 again with 2 N NaOH solution, and the mixture was stirred at room temperature for 16 h. The mixture was subjected to rotary evaporation to dryness, and acetic acid (10 mL) was added to the residue. The system was heated to 100°C and reacted for 20 hours, then subjected to rotary evaporation to dryness. The crude product was purified by macroporous resin XAD 1600 column chromatography to afford compound 4f (2.25 g, yield: 72.8%).
MS (ESI): m/z 782.1 $[M+1]^+$.

Step 6) Preparation of compound 4

SM-3 (816 mg, 8.96 mmol, 4.0 eq) was placed in a 50 mL single-necked flask, and deionized water (5 mL) was added. The mixture was stirred for dissolution. The pH of the resulting solution was adjusted to 7 with 4 N HCl. Compound 4f (1.75 g, 2.24 mmol, 1.0 eq), HOBT (151 mg, 1.12 mmol, 0.5 eq) and EDCI (2.15 g, 11.2 mmol, 5.0 eq) were added to the reaction system separately, and the mixture was stirred at room temperature for 3 days. The product was purified by preparative liquid chromatography to afford compound 4 (1.72 g, yield: 77.8%).
MS (ESI): m/z 1001.3 $[M+1]^+$.

Example 5

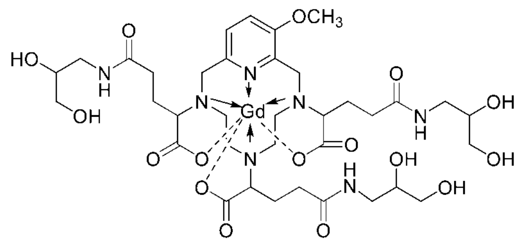

2,2',2'-(1$^3$-methoxy-3,6,9-triaza-1(2,6)-pyridine cyclodecane-3,6,9-triyl)tris(5-((2,3-dihydroxypropyl)amino)-5-oxopentanedioic acid)gadolinium (III) complex (compound 5)

Step 1) Preparation of 2,6-bis(bromomethyl)-3-methoxypyridine (compound 5b):

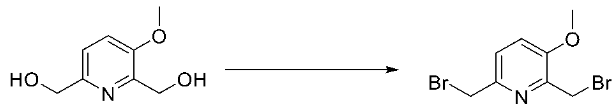

**5a** **5b**

In a 250 mL single-necked flask, compound 5a (3.07 g, 18.15 mmol) was added and completely dissolved in $CHCl_3$ (90 mL). Then, phosphorus tribromide (22.10 g, 81.66 mmol, 7.68 mL) was slowly added dropwise in an ice bath. After the addition was completed, the reaction system was refluxed at 60°C overnight. At 0°C, about 155 mL of saturated sodium carbonate aqueous solution (1.6 M) was slowly added to the solution. The mixture was adjusted to pH = 8 with saturated sodium bicarbonate solution, stirred for about 30 min, and the layers were separated. The aqueous phase was extracted with dichloromethane (50 mL × 3). The combined organic phases were washed with water (25 mL × 2), dried over anhydrous sodium sulphate, stirred for 30 min, and subjected to rotary evaporation under reduced pressure to dryness to obtain 3.44 g of a pink solid (purity: 96%, yield: 64.27%).

Step 2) Preparation of compound 5

With reference to the method described in example 4, compound 5 (purity: 97.84%) was synthesised using 2,6-bis(bromomethyl)-3-methoxypyridine (compound 5b) as a starting material.
MS (ESI): m/z 1001.3 $[M+1]^+$.

Example 6

Compound **4f** was prepared with reference to the method described in example 4, and further purified by preparative HPLC (XP tC18, 250 mm × 50 mm×7 μm, detection wavelength: 214 nm, flow rate: 100 ml/min, mobile phase: 0.1% aqueous acetic acid solution (mobile phase A)/acetonitrile (mobile phase B), elution gradient: 92:8→40:60→92:8). Four signal peaks were obtained, corresponding to different pairs of enantiomers.
Retention times were 33.48 min, 33.98 min, 34.75 min and 39.55 min, respectively, wherein the peak with a retention time of 39.55 min for the RRR/SSS enantiomer pair

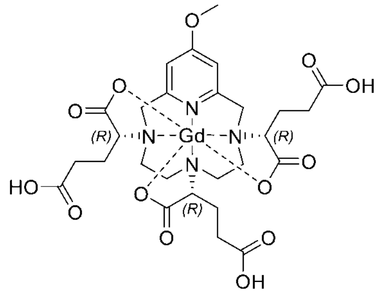
/
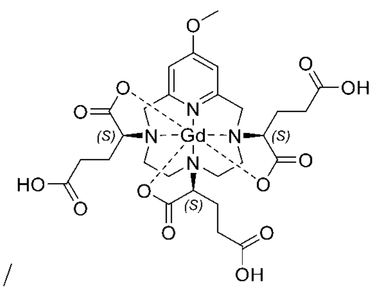
.

Step 1)

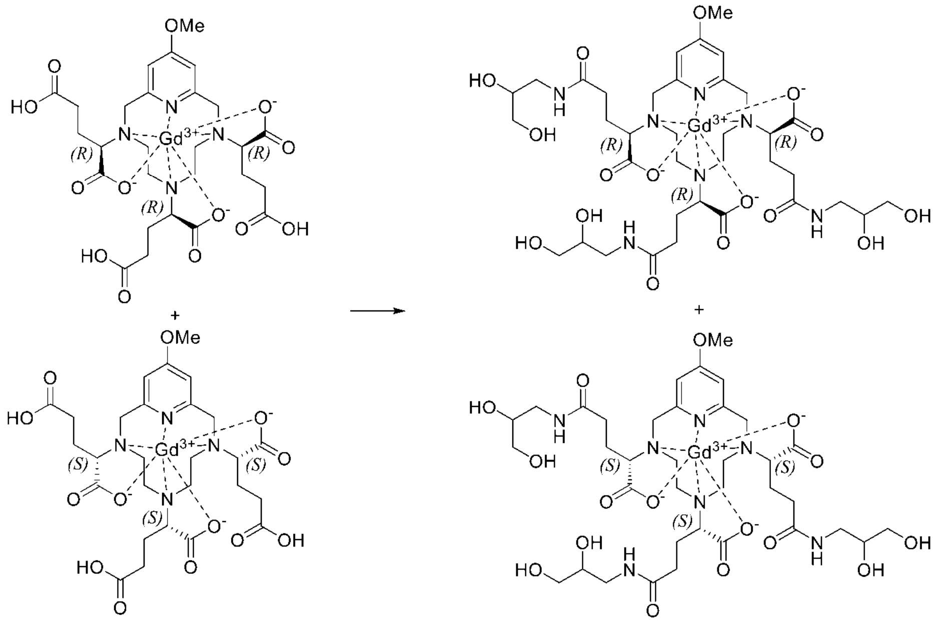

With reference to step 4) of example 4, the enriched RRR/SSS enantiomer pair was used to replace compound **4f,** and then reacted with isoserinol (3-amino-1,2-propanediol) to afford the RRR/SSS enantiomer

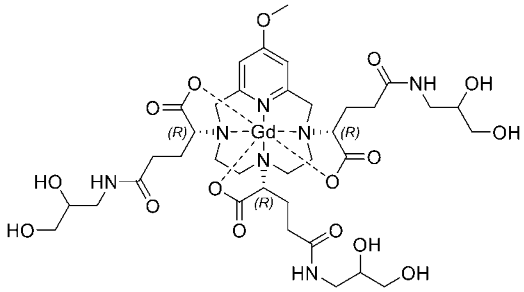
/
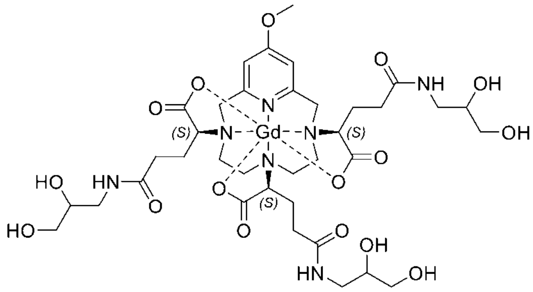

.

MS (ESI): m/z 782.14 $[M+H]^+$.

Test example 1: Relaxivity of the compound in water

1. Test sample

Gadobutrol, RC1 (Gadopiclenol, prepared according to the method described in example 2 of WO 2020030618 A1), compound 4 and compound 5.

2. Experiment instruments

Niumag (Suzhou) Nuclear Magnetic Resonance Contrast Agent Relaxivity Analyser with a 0.5 T magnetic field
Bruker Small Animal Magnetic Resonance Imaging System (BioSpec 70/20 USR) with a 7 T magnetic field

3. Experimental method

Gadobutrol, RC1, compound 4 and compound 5 were accurately weighed and dissolved in deionized water to prepare solutions with concentrations of 0.5 mM, 1 mM, 2 mM and 4 mM, respectively. 1 mL of the test sample was placed in a 1.5 mL EP (eppendorf) tube, and the longitudinal relaxation time T1 and transverse relaxation time T2 under different magnetic field conditions were measured separately using the Niumag (Suzhou) Nuclear Magnetic Resonance Contrast Agent Relaxivity Analyser with a 0.5 T magnetic field. The test sample was loaded into a 0.2 mL sample tube until full, and the longitudinal relaxation time T1 and transverse relaxation time T2 under different magnetic field conditions were measured separately using the Bruker Small Animal Magnetic Resonance Imaging System (BioSpec 70/20 USR) with a 7 T magnetic field.

Based on the relaxation times T1 and T2 of samples at different concentrations, the relaxivities $r_1$ and $r_2$ were calculated respectively. The relaxivity was calculated according to the following equation:

$$r = \frac{1}{\Delta T}/C,$$

r: relaxivity; T: relaxation time (s); C: contrast agent concentration (mM).

4. Experimental results

Table 1

| Name | Relaxivity ($r_1/r_2$) $mM^{-1}$ $s^{-1}$ 0.5 T | Relaxivity ($r_1/r_2$) $mM^{-1}$ $s^{-1}$ 7 T |
|---|---|---|
| Gadobutrol | 4.4/5.8 | 3.5/5.6 |
| RC1 | 11.7/16.3 | 6.3/17.3 |
| Compound 4 | 15.5/20.8 | 10.7/22.0 |
| Compound 5 | 9.3/11.5 | 5.2/11.5 |

Test example 2: Kinetic stability of compounds in acidic medium

1. Test sample

Gadobutrol, RC1 (Gadopiclenol, prepared according to the method described in example 2 of WO 2020030618 A1), compound 4 and compound 5.

2. Experiment instruments

Molecular Devices SpectraMax M2/M2e Microplate Reader.

3. Experimental method

(1) Hydrochloric acid (pH 1.2) was used as a solvent, and solutions of RC1, compound 4, compound 5 and gadobutrol were prepared at a concentration of $8 \times 10^{-6}$ M, respectively, for subsequent use.
(2) Standard curve plotting: An appropriate amount of $GdCl_3 \cdot 6H2O$ (Shanghai Dingxian Biotechnology Co., Ltd.) was accurately weighed into vials. Then, 1 mL of solutions containing free $Gd^{3+}$ at concentrations of 0, $1 \times 10^{-6}$ M, $2 x 10^{-6}$ M, $4 \times 10^{-6}$ M, $6 \times 10^{-6}$ M, $8 \times 10^{-6}$ M and $10 \times 10^{-6}$ M were prepared respectively using deionized water. Each of the solutions was thoroughly mixed with 40 μL of $5.3 \times 10^{-4}$ M arsenazo III solution (a chromogenic reagent, Shanghai

Macklin Biochemical Co., Ltd.) via vortexing. After incubation in the dark at room temperature for 15 min, 150 μL of the mixture was transferred to each well (with duplicate wells) in a 96-well plate, and the absorbance was determined at a wavelength of 655 nm. A standard curve of $Gd^{3+}$ concentration versus absorbance was established.

(3) Compound testing: 500 ml of each pre-prepared test solution at a concentration of $8 \times 10^{-6}$ M was evenly dispensed into 3 vials, with 150 mL per vial. The vials were placed at 37°C. 25 mL of the solution was collected from each vial at 0 h, 24 h, 48 h, 96 h and 168 h, respectively. NaOH was added to adjust the pH of the solution to neutral (as indicated by pH test paper). Subsequently, 1 mL of $5.3 \times 10^{-4}$ M arsenazo III solution was added, and the mixture was thoroughly mixed via vortexing. After incubation in the dark at room temperature for 15 min, 150 μL of the mixture was transferred to each well (with duplicate wells) in a 96-well plate, and the absorbance of the samples was determined at a wavelength of 655 nm.

(4) The concentration of free $Gd^{3+}$ and the dissociation rate of each test solution at different time points were calculated based on the formula fitted from the standard curve.

4. Experimental results

Table 2

| Compound | | $4^{a, b}$ | 5 | RC1 | Gadobutrol |
|---|---|---|---|---|---|
| $Gd^{3+}$ Dissociation rate (%) | 0 h | BLQ | BLQ | BLQ | BLQ |
| | 24 h | BLQ | 24.1 | 24.7 | 73.5 |
| | 48h | BLQ | 28.7 | 35.6 | 70.1 |
| | 96 h | 0.59 | 32.3 | 37.8 | 64.6 |
| | 168 h | 23.9 | 42.1 | 61.1 | 95.3 |

Notes: BLQ is below the limit of quantification, a: $P < 0.0001$ vs. RC1, b: $P < 0.0001$ vs. gadobutrol.

Test example 3: Determination of relaxivity of compounds in human plasma 1. Test sample

Gadobutrol (Gadovist®), *Elucirem*™ (Gadopiclenol, commercially purchased), compound 4.

2. Experiment instruments

Medium-Sized Nuclear Magnetic Resonance Imaging Analyser (model: MesoMR23-060-I) with a 0.5 T magnetic field

3. Experimental method

Human plasma containing sodium heparin was used as the matrix. Compound 4 (0.5 M), gadobutrol injection (1 M) and Gadopiclenol injection (0.5 M) were separately diluted to prepare test solutions at concentrations of 4, 2, 1, 0.5 and 0.25 mM, respectively. 1 mL of each test sample was transferred into a 1.5 mL centrifuge tube, and the longitudinal relaxation time T1 and transverse relaxation time T2 were measured at a 0.5 T magnetic field strength using the Medium-Sized Nuclear Magnetic Resonance Imaging Analyser (MesoMR23-060-I).

Based on the relaxation times T1 and T2 of samples at different concentrations, the relaxivities $r_1$ and $r_2$ were calculated respectively. The relaxivity was calculated according to the following equation:

$$r = \frac{1}{\Delta T}/C,$$

r: relaxivity; T: relaxation time (s); C: contrast agent concentration (mM).

4. Experimental results

Table 3

| Name | Relaxivity ($r_1/r_2$) $mM^{-1}$ $s^{-1}$ 0.5 T |
|---|---|
| Gadobutrol | 7.5/8.1 |
| *Elucirem*™ | 12.7/17.8 |
| Compound 4 | 16.0/20.1 |

Test example 4: Metal transfer stability test of compounds in the presence of zinc ions

1 . Experimental protocol
2. Test samples: compound 4, gadodiamide, gadobutrol.
3. Experiment instruments: VTMR20-010V-I Nuclear Magnetic Resonance Cross-Linking Density Imaging Analyser
4. Experimental method

A 2.5 mM solution of contrast agent solutions and a 2.5 mM solution of zinc chloride were respectively prepared in phosphate buffer (0.026 mol/L $KH_2PO_4$ and 0.041 mol/L $Na_2HPO_4$). An equal volume of a 2.5 mM $ZnCl_2$ solution was added to the a 2.5 mM gadolinium contrast agent solution, and the mixture was mixed by shaking. This moment of complete mixing was designated as time zero (t = 0). Immediately thereafter, a 0.5 mL aliquot was withdrawn and subjected to measurement of the longitudinal relaxation time, $T1_{(0)}$, at 37°C and a magnetic field strength of 0.5 T. The mixed solution was then incubated at 37°C. 0.5 mL of the solution was taken at time points of 5 min, 180 min, 1440 min and 5760 min, respectively, to determine $T1_{(t)}$ at different time points. The ratio of the relaxivity at different time points to the initial relaxivity at time 0, namely $R_{1(t)}/R_{1(0)}$, was calculated.

5. Experimental results

Table 4

| Time (min) | Gadodiamide | Gadobutrol | Compound 4 |
|---|---|---|---|
| | $R_{1(t)}/R_{1(0)}$ | $R_{1(t)}/R_{1(0)}$ | $R_{1(t)}/R_{1(0)}$ |
| 0 | 1 | 1 | 1 |
| 5 | 1.07 | 0.93 | 0.77 |
| 180 | 0.59 | 0.82 | 0.91 |
| 1440 | 0.59 | 0.96 | 0.89 |
| 5760 | 0.38 | 0.90 | 1.05 |

Test example 5: In vitro canine plasma protein binding rate of compounds

1. Test samples: *Elucirem*™ (Gadopiclenol, commercially purchased, lot P233A), compound 4
2. Experimental method

*Elucirem*™ and compound 4 were respectively diluted with blank canine plasma to prepare plasma samples at a concentration of 1 mM. The binding rates of Gadopiclenol and compound 4 to canine plasma proteins were determined by ultracentrifugation. After the preparation of plasma samples, 0.5 mL of plasma samples were transferred into ultracentrifugation tubes, with 3 replicates per concentration. After incubation in a $CO_2$ incubator at 37°C for 0.5 h, the samples were subjected to ultracentrifugation at 627,000 g for 3 hours at 37°C. After centrifugation, 100 μL of samples were taken from the central layer of the ultracentrifugation tubes and mixed with 100 μL of blank plasma. The samples were stored at -70°C for subsequent determination. The concentrations of Gadopiclenol and compound 4 in the samples were quantitatively analysed by LC-MS/MS.

The plasma protein binding rates of analytes at different concentration levels were calculated using the following formulas:

Free rate% = (Concentration of the central layer in ultracentrifugation tubes ($C_u$)/Concentration of non-ultracentrifuged samples after 0.5 h incubation ($C_{0.5h}$))*100%,

$$\text{Binding rate\%} = 100\% - \text{Free rate\%}.$$

3. Experimental results:

The binding rates of Gadopiclenol and compound 4 to canine plasma proteins are shown in Table 5. Gadopiclenol does not bind to plasma proteins, whereas compound 4 exhibits an average plasma protein binding rate of 19.6%, suggesting a potential enhancement effect on plasma relaxivity.

| | Binding rate% | Average% |
|---|---|---|
| Gadopiclenol | -1.27<br>-2.05<br>-0.41 | 0* |
| Compound 4 | 16.8<br>18.7<br>23.4 | 19.6 |

Notes: Due to detection errors, if the binding rate is less than 0, the average value is recorded as 0%.
Test example 6: General observation test of compounds administered intravenously to rats

1. Test samples: *Elucirem*™ (Gadopiclenol, commercially purchased, lot P233A), compound 4
2. Experimental method

SD rats were given a single intravenous injection of *Elucirem*™ or compound 4 at a dose of 6 mmol/kg, with 8 animals per group and equal numbers of males and females. All animals were subjected to detailed clinical observations during the test. The observation items included the administration site, skin, coat, face, eyes, ears, nose, oral cavity, chest, abdomen, urogenital region, limbs and other body parts, as well as respiration, movement, urination, defecation and behavioural changes.

3. Experimental results

After administration of *Elucirem*™, all 8 animals immediately exhibited redness of the four paws/both ears, and 5 of them also exhibited swelling of the forepaws/four paws. After administration of compound 4, 5 out of 8 animals immediately exhibited redness of the four paws/both ears, and 4 of them also exhibited swelling of the forepaws/four paws; while 3 animals exhibited no abnormalities.

## Claims

1. A compound as shown in formula I or a pharmaceutically acceptable salt thereof

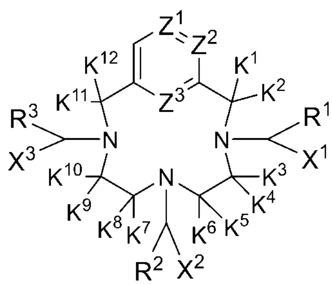

,

**characterised in that** $R^1$, $R^2$ and $R^3$ are each independently selected from - COOH, $-P(O)(OH)_2$ or $-P(O)(R^4)(OH)$, and $R^4$ is selected from hydrogen or $C_{1-4}$ alkyl;
$X^1$, $X^2$ and $X^3$ are each independently selected from L-Y, wherein L is selected from substituted or unsubstituted $C_{1-6}$ alkylene, and Y is selected from - COOH, $-CONH_2$, $-CONR^5R^6$ or $-NR^7COR^8$;
$R^5$ is selected from hydrogen, $C_{1-6}$ alkyl or $C_{1-6}$ hydroxylalkyl, $R^6$ is selected from $C_{1-6}$ alkyl or $C_{1-6}$ hydroxylalkyl, and at least one of $R^5$ and $R^6$ is $C_{1-6}$ hydroxyalkyl;
$R^7$ is selected from hydrogen, $C_{1-6}$ alkyl or $C_{1-6}$ hydroxylalkyl, $R^8$ is selected from $C_{1-6}$ alkyl or $C_{1-6}$ hydroxylalkyl, and at least one of $R^7$ and $R^8$ is $C_{1-6}$ hydroxyalkyl;
$Z^1$ is $CR^9$;
$Z^2$ is $CR^{10}$;
$R^9$ and $R^{10}$ are independently selected from hydrogen, halogen, nitro, amino, cyano, $C_{1-6}$ alkyl, $C_{1-6}$ alkoxy, 3- to 6-membered cycloalkyl, 3- to 6-membered cycloalkoxy, 3- to 6-membered heterocycloalkyl, 3- to 6-membered heterocycloalkoxy, 6-membered aryl or 5- to 6-membered heteroaryl, wherein the alkyl, alkoxy, cycloalkyl, heterocycloalkyl, cycloalkoxy, heterocyclyloxy, aryl or heteroaryl is optionally substituted with one or more $R^{1A}$, each of which is independently selected from halogen, hydroxyl, oxo, nitro, cyano, amino, $C_{1-6}$ alkyl or $C_{1-6}$ alkoxy, and $R^9$ and $R^{10}$are not both hydrogen,
or $R^9$ and $R^{10}$, together with adjacent carbon atoms, form 6-membered aryl or 5- to 6-membered heteroaryl, wherein the aryl or heteroaryl is optionally substituted with one or more $R^{1B}$, each of which independently selected from halogen, hydroxyl, nitro, cyano, amino, substituted or unsubstituted $C_{1-6}$ alkyl, or substituted or unsubstituted $C_{1-6}$ alkoxy;
$Z^3$ is selected from CH or N;
$K^1$-$K^{12}$ are independently selected from hydrogen, $C_{1-6}$ alkyl or $C_{1-6}$ hydroxylalkyl;
or $K^1$ and $K^2$ form a 3- to 6-membered substituted or unsubstituted carbocyclic ring, or $K^3$ or $K^4$ forms a 3- to 6-membered substituted or unsubstituted carbocyclic ring with $K^5$ or $K^6$, or $K^7$ or $K^8$ forms a 3- to 6-membered substituted or unsubstituted carbocyclic ring with $K^9$or $K^{10}$, or $K^{11}$ and $K^{12}$ form a 3- to 6-membered substituted or unsubstituted carbocyclic ring.

2. The compound or the pharmaceutically acceptable salt thereof according to claim 1, **characterised in that** $R^1$, $R^2$ and $R^3$ are -COOH.

3. The compound or the pharmaceutically acceptable salt thereof according to claim 1 or 2, **characterised in that** $Z^3$ is N.

4. The compound or the pharmaceutically acceptable salt thereof according to any one of claims 1-3, **characterised in that** $Z^1$ is $CR^9$, and $R^9$ is selected from halogen, $C_{1-6}$ alkyl, $C_{1-6}$ alkoxy, 6-membered aryl or 5- to 6-membered heteroaryl, wherein the alkyl, alkoxy, aryl or heteroaryl is optionally substituted with one or more $R^{1A}$ as defined in claim 1; further, $R^9$ is preferably chlorine, fluorine, methoxy, ethoxy or phenyl.

5. The compound or the pharmaceutically acceptable salt thereof according to claim 1 or 4, **characterised in that** the compound as shown in formula I is

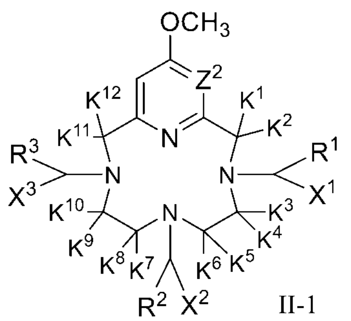

II-1

wherein $R^1$-$R^3$, $Z^2$, $K^1$-$K^{12}$, and $X^1$-$X^3$are as defined in claim 1.

6. The compound or the pharmaceutically acceptable salt thereof according to any one of claims 1-5, **characterised in that** $Z^2$ is $CR^{10}$, and $R^{10}$ is selected from hydrogen, halogen, $C_{1-6}$ alkyl, $C_{1-6}$ alkoxy, 6-membered aryl or 5- to 6-membered heteroaryl, wherein the alkyl, alkoxy, aryl or heteroaryl is optionally substituted with one or more $R^{1A}$ as defined in claim 1; further, $R^{10}$ is preferably hydrogen, chlorine, fluorine, methoxy, ethoxy or phenyl.

**7.** The compound or the pharmaceutically acceptable salt thereof according to any one of claims 1-3, **characterised in that** $R^9$ and $R^{10}$, together with adjacent carbon atoms, form 6-membered aryl, the aryl is optionally substituted with one or more $R^{1B}$ as defined in claim 1.

**8.** The compound or the pharmaceutically acceptable salt thereof according to claim 1 or 7, **characterised in that** the compound as shown in formula I is

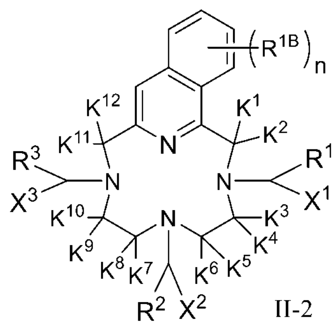

II-2 ,

wherein n is an integer from 0 to 4; $R^1$-$R^3$, $K^1$-$K^{12}$, $X^1$-$X^3$ and $R^{1B}$ are as defined in claim 1.

**9.** The compound or the pharmaceutically acceptable salt thereof according to any one of claims 1-8, **characterised in that** $X^1$, $X^2$ and $X^3$ are each independently selected from L-Y, wherein L is selected from substituted or unsubstituted $C_{1-3}$ alkylene, and Y is selected from -COOH or $-CONH_2$.

**10.** The compound or the pharmaceutically acceptable salt thereof according to any one of claims 1-9, **characterised in that** $X^1$, $X^2$ and $X^3$ are each independently selected from L-Y, wherein L is selected from substituted or unsubstituted $C_{1-3}$ alkylene, and Y is selected from $-CONR^5R^6$ or $-NR^7COR^8$ $R^5$ is selected from hydrogen or methyl, $R^6$ is $C_{1-6}$ hydroxylalkyl, $R^7$ is selected from hydrogen or methyl, and $R^8$ is $C_{1-6}$ hydroxylalkyl.

**11.** The compound or the pharmaceutically acceptable salt thereof according to claim 1 or 10, **characterised in that** $R^6$ is $C_{2-6}$ hydroxylalkyl, preferably selected from $-CH_2CH_2OH$, $-CH(OH)CH_2OH$, $-CH(CH_2OH)_2$, - $CH_2(CHOH)_pCH_2OH$ or $-C(CH_2OH)_3$, wherein p is an integer from 1 to 4.

**12.** The compound or the pharmaceutically acceptable salt thereof according to claim 1 or 10, **characterised in that** $R^8$ is $C_{2-6}$ hydroxylalkyl, preferably selected from $-CH_2CH_2OH$, $-CH(OH)CH_2OH$, $-CH(CH_2OH)_2$, - $CH_2(CHOH)_pCH_2OH$ or $-C(CH2OH)_3$, wherein p is an integer from 1 to 4.

**13.** The compound or the pharmaceutically acceptable salt thereof according to claim 1 or 10, **characterised in that** $X^1$, $X^2$ and $X^3$ are each independently selected from L-Y, wherein L is selected from substituted or unsubstituted $C_{1-3}$ alkylene, and Y is $-CONR^5R^6$, $R^5$ is hydrogen, $R^6$ is selected from $-CH_2CH_2OH$, $-CH(OH)CH_2OH$, $-CH(CH_2OH)_2$, $-CH_2(CHOH)_pCH_2OH$ or - $C(CH2OH)_3$, and p is an integer from 1 to 4.

**14.** An isotopic substitute of the compound according to any one of claims 1-13, **characterised in that** preferably, the isotopic substitute is a deuterated compound.

**15.** A multimer of the compound according to any one of claims 1-13, **characterised in that** preferably, the multimer is a dimer or trimer.

**16.** A complex of the compound according to any one of claims 1-13, or the deuterated compound as defined in claim 14, or the multimer according to claim 15 and M, **characterised in that** M is $Gd^{3+}$, $Mn^{2+}$ or $Fe^{3+}$.

**17.** The complex according to claim 16, **characterised in that** the complex is selected from:

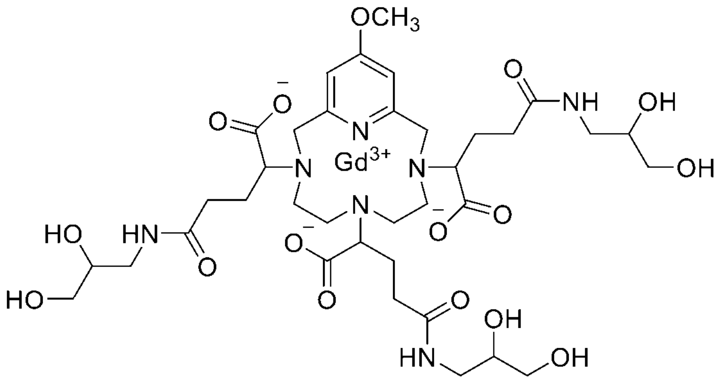

,

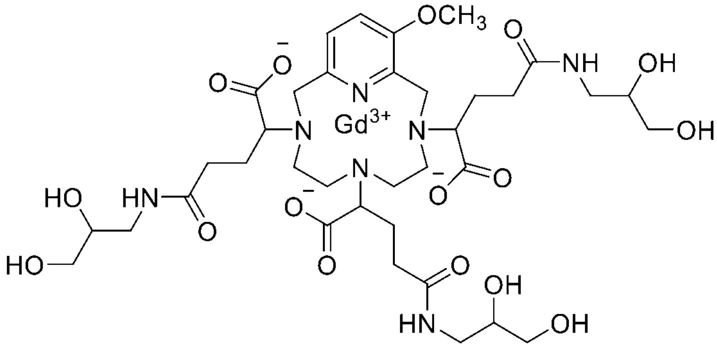

,

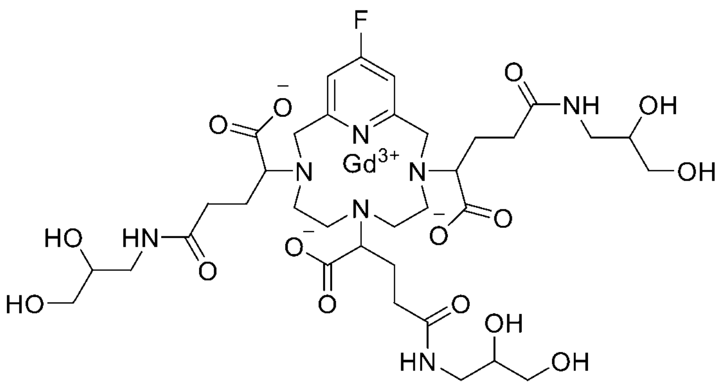

,

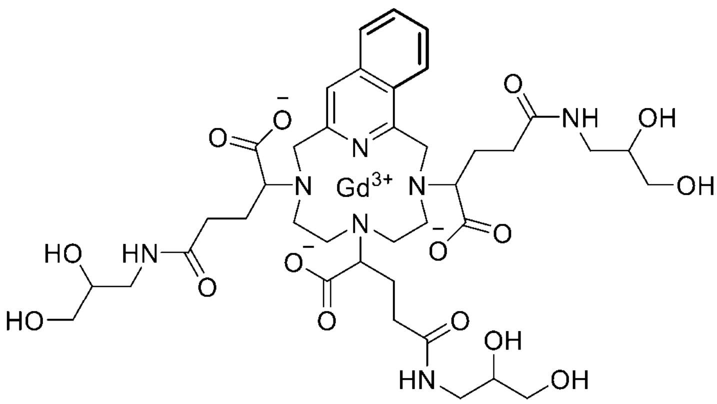

,

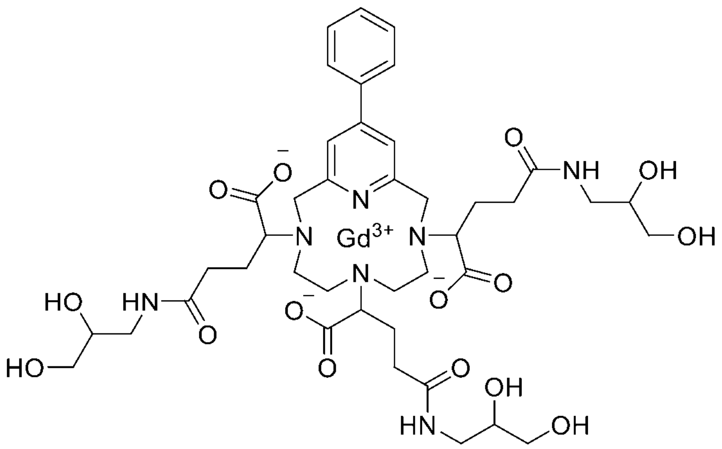

,

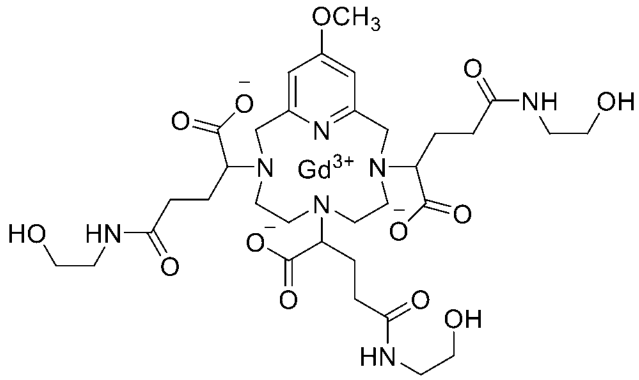

,

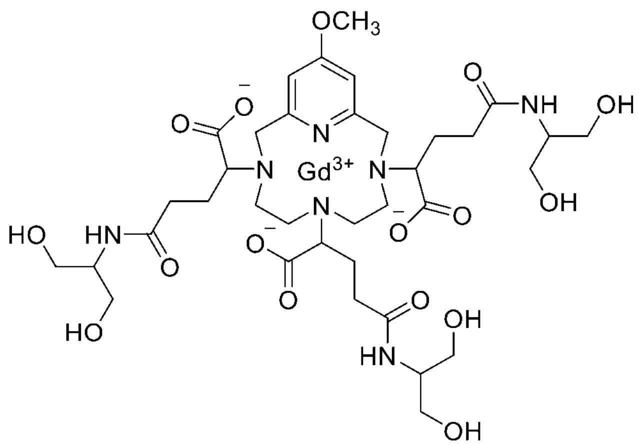

,

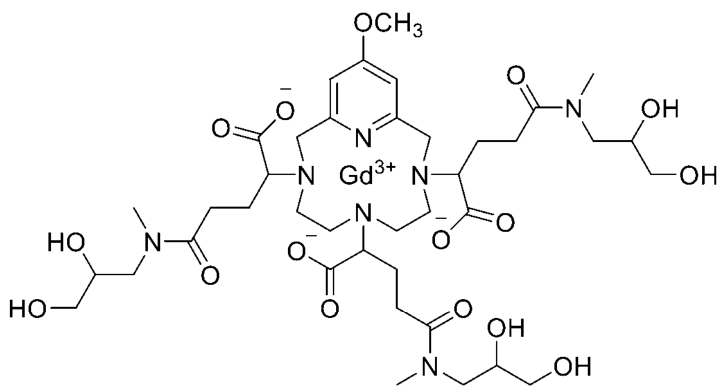

or

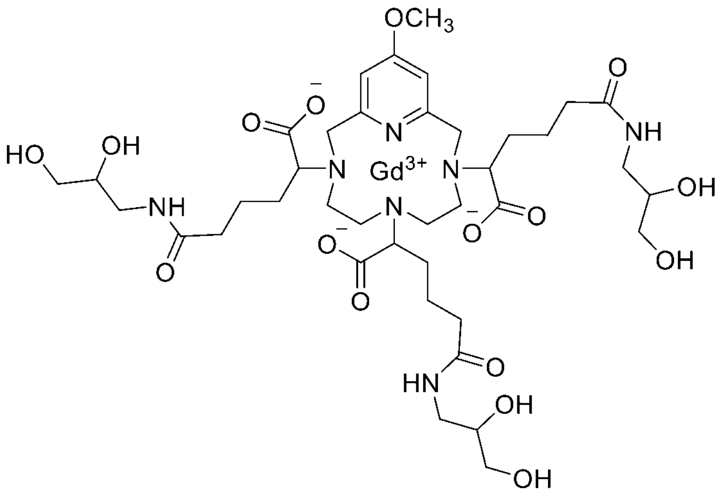

.

18. A pharmaceutical composition, **characterised by** comprising the compound according to any one of claims 1-13, or the deuterated compound as defined in claim 14, or the multimer according to claim 15 or the complex according to claim 16 or 17.

19. Use of the compound according to any one of claims 1-13, or the deuterated compound as defined in claim 14, or the multimer according to claim 15, or the complex according to claim 16 or 17 or the composition according to claim 18 in the preparation of a drug for nuclear magnetic resonance imaging.

**INTERNATIONAL SEARCH REPORT**

International application No.
**PCT/CN2024/115680**

**A. CLASSIFICATION OF SUBJECT MATTER**

C07D471/08(2006.01)i; C07D487/04(2006.01)i; C07D257/00(2006.01)i; A61K51/04(2006.01)i

According to International Patent Classification (IPC) or to both national classification and IPC

**B. FIELDS SEARCHED**

Minimum documentation searched (classification system followed by classification symbols)

IPC:C07D,A61K

Documentation searched other than minimum documentation to the extent that such documents are included in the fields searched

Electronic data base consulted during the international search (name of data base and, where practicable, search terms used)

CNTXT, ENTXT, CNKI, ISI Web of Knowledge, CAPLUS (STN), REGISTRY(STN): 大环, 钆, 螯合, 核磁共振, chelate, Gd, macrocyc+, MRI

**C. DOCUMENTS CONSIDERED TO BE RELEVANT**

| Category* | Citation of document, with indication, where appropriate, of the relevant passages | Relevant to claim No. |
|---|---|---|
| X | US 2009214441 A1 (GUERBET S.A.) 27 August 2009 (2009-08-27) claims 1-11, and description, paragraph [0242] | 1-14, 16-19 |
| Y | US 2009214441 A1 (GUERBET S.A.) 27 August 2009 (2009-08-27) claims 1-11, and description, paragraph [0242] | 15 |
| Y | CN 101305006 A (GUERBET S.A.) 12 November 2008 (2008-11-12) claims 1-15, and description, page 25, paragraph 4, and page 34, embodiment 7 | 15 |
| A | CN 112533921 A (BRACCO IMAGING SPA.) 19 March 2021 (2021-03-19) claims 7-9 and 18, and description, paragraphs [0280], [0314], and [0329] | 1-19 |
| A | CN 1354751 A (GUERBET S.A.) 19 June 2002 (2002-06-19) claims 1 and 16-18, and description, page 6, paragraph 3 | 1-19 |
| A | US 2020338217 A1 (GENERAL ELECTRIC COMPANY) 29 October 2020 (2020-10-29) abstract, claim 27, and description, paragraphs [0008]-[0022] | 1-19 |
| A | CN 116209661 A (GABAI FRANCE) 02 June 2023 (2023-06-02) description, paragraphs [0025]-[0026], [0055], [0060]-[0061], and [0146] | 1-19 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 November 2024** | **25 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/115680**

| Patent document cited in search report | Publication date (day/month/year) | Patent family member(s) | Publication date (day/month/year) |
|---|---|---|---|
| US 2009214441 A1 | 27 August 2009 | FR 2883562 A1 | 29 September 2006 |
| | | FR 2883562 B1 | 27 February 2009 |
| | | US 8268810 B2 | 18 September 2012 |
| | | DE 602006008135 D1 | 10 September 2009 |
| | | ES 2330140 T3 | 04 December 2009 |
| | | EP 1885721 A2 | 13 February 2008 |
| | | EP 1885721 B1 | 29 July 2009 |
| | | WO 2006100305 A2 | 28 September 2006 |
| | | WO 2006100305 A3 | 09 November 2006 |
| | | ATE 437878 T1 | 15 August 2009 |
| CN 101305006 A | 12 November 2008 | EP 1931673 A1 | 18 June 2008 |
| | | EP 1931673 B1 | 29 August 2012 |
| | | CA 2625207 A1 | 19 April 2007 |
| | | CA 2625207 C | 13 May 2014 |
| | | PL 1931673 T3 | 30 November 2012 |
| | | DK 1931673 T3 | 08 October 2012 |
| | | JP 2009513574 A | 02 April 2009 |
| | | JP 5317270 B2 | 16 October 2013 |
| | | PT 1931673 E | 24 September 2012 |
| | | WO 2007042506 A1 | 19 April 2007 |
| | | JP 2013209400 A | 10 October 2013 |
| | | JP 5731572 B2 | 10 June 2015 |
| | | KR 20140060565 A | 20 May 2014 |
| | | KR 101440761 B1 | 17 September 2014 |
| | | US 2009169479 A1 | 02 July 2009 |
| | | US 8114863 B2 | 14 February 2012 |
| | | EP 2457914 A1 | 30 May 2012 |
| | | EP 2457914 B1 | 06 August 2014 |
| | | NL 301259 I2 | 25 March 2024 |
| | | KR 20080080495 A | 04 September 2008 |
| | | ES 2519369 T3 | 06 November 2014 |
| | | FIC 20240003 I1 | 26 February 2024 |
| | | FR 2891830 A1 | 13 April 2007 |
| | | FR 2891830 B1 | 24 June 2011 |
| | | BRPI 0617151 A2 | 12 July 2011 |
| | | BRPI 0617151 B1 | 11 August 2020 |
| | | BRPI 0617151 B8 | 27 July 2021 |
| | | FR 24C1006 I1 | 19 April 2024 |
| | | ES 2390092 T3 | 06 November 2012 |
| CN 112533921 A | 19 March 2021 | US 2020376145 A1 | 03 December 2020 |
| | | US 10973934 B2 | 13 April 2021 |
| | | US 2021196840 A1 | 01 July 2021 |
| | | US 12036289 B2 | 16 July 2024 |
| | | ES 2841070 T3 | 07 July 2021 |
| | | DK 3770160 T3 | 20 February 2023 |
| | | EP 3687994 A1 | 05 August 2020 |
| | | EP 3687994 B1 | 04 November 2020 |
| | | PL 3770160 T3 | 29 May 2023 |
| | | PT 3770160 T | 21 February 2023 |
| | | JP 2021533165 A | 02 December 2021 |

Form PCT/ISA/210 (patent family annex) (July 2022)

**INTERNATIONAL SEARCH REPORT**
**Information on patent family members**

International application No.
**PCT/CN2024/115680**

| Patent document cited in search report | Publication date (day/month/year) | Patent family member(s) | Publication date (day/month/year) |
|---|---|---|---|
| | | JP 7558146 B2 | 30 September 2024 |
| | | EP 3770160 A1 | 27 January 2021 |
| | | EP 3770160 B1 | 23 November 2022 |
| | | ES 2938015 T3 | 03 April 2023 |
| | | AU 2019319599 A1 | 28 January 2021 |
| | | AU 2019319599 B2 | 04 January 2024 |
| | | BR 112021001102 A2 | 20 April 2021 |
| | | BR 112021001102 B1 | 20 December 2022 |
| | | CA 3105610 A1 | 13 February 2020 |
| | | CA 3105610 C | 28 May 2024 |
| | | DK 3687994 T3 | 25 January 2021 |
| | | FI 3770160 T3 | 16 March 2023 |
| | | PL 3687994 T3 | 04 May 2021 |
| | | KR 20210041566 A | 15 April 2021 |
| | | EP 4151637 A1 | 22 March 2023 |
| | | WO 2020030618 A1 | 13 February 2020 |
| | | PT 3687994 T | 07 December 2020 |
| CN 1354751 A | 19 June 2002 | HUP 0201468 A2 | 28 August 2002 |
| | | HUP 0201468 A3 | 28 January 2010 |
| | | HU 227966 B1 | 30 July 2012 |
| | | WO 0075141 A1 | 14 December 2000 |
| | | JP 2003501430 A | 14 January 2003 |
| | | JP 4719391 B2 | 06 July 2011 |
| | | IL 146304 A | 10 April 2006 |
| | | AU 5540400 A | 28 December 2000 |
| | | CA 2376497 A1 | 14 December 2000 |
| | | CA 2376497 C | 20 October 2009 |
| | | FR 2794744 A1 | 15 December 2000 |
| | | FR 2794744 B1 | 21 September 2001 |
| | | MXPA 01011439 A | 04 June 2002 |
| | | DE 60001564 D1 | 10 April 2003 |
| | | DE 60001564 T2 | 11 December 2003 |
| | | KR 20020008218 A | 29 January 2002 |
| | | PL 352483 A1 | 25 August 2003 |
| | | PL 216974 B1 | 30 May 2014 |
| | | ATE 233762 T1 | 15 March 2003 |
| | | ES 2188556 T3 | 01 July 2003 |
| | | TR 200103525 T2 | 21 May 2002 |
| | | IL 146304 A0 | 25 July 2002 |
| | | US 6440956 B1 | 27 August 2002 |
| | | EP 1183255 A1 | 06 March 2002 |
| | | EP 1183255 B1 | 05 March 2003 |
| | | PT 1183255 E | 30 June 2003 |
| | | BR 0011436 A | 05 March 2002 |
| | | NO 20015991 D0 | 07 December 2001 |
| | | NO 20015991 L | 06 February 2002 |
| | | NO 321121 B1 | 20 March 2006 |
| | | RU 2232763 C2 | 20 July 2004 |
| | | DK 1183255 T3 | 02 June 2003 |
| US 2020338217 A1 | 29 October 2020 | US 11110186 B2 | 07 September 2021 |

Form PCT/ISA/210 (patent family annex) (July 2022)

**INTERNATIONAL SEARCH REPORT**
**Information on patent family members**

International application No.
**PCT/CN2024/115680**

| Patent document cited in search report | Publication date (day/month/year) | Patent family member(s) | Publication date (day/month/year) |
|---|---|---|---|
| | | JP 2021506901 A | 22 February 2021 |
| | | JP 7332600 B2 | 23 August 2023 |
| | | EP 3728168 A1 | 28 October 2020 |
| | | EP 3728168 B1 | 30 March 2022 |
| | | WO 2019122255 A1 | 27 June 2019 |
| CN 116209661 A | 02 June 2023 | WO 2022013454 A1 | 20 January 2022 |
| | | US 2023339945 A1 | 26 October 2023 |
| | | KR 20230041007 A | 23 March 2023 |
| | | EP 4182314 A1 | 24 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007042506 A
- WO 1993011800 A
- WO 2000075141 A
- WO 2003074523 A
- EP 0438206 A
- WO 2006100305 A
- WO 2018115314 A
- WO 2019122255 A
- US 5403572 A
- EP 0661279 A
- US 20200353105 A
- US 5334371 A
- WO 2004112839 A
- WO 2005049005 A
- WO 2005049095 A
- WO 2005042033 A
- WO 20019188 A
- CN 112533921 A
- WO 2020030618 A1


### Non-patent literature cited in the description


- *Bioorganic and Medicinal Chemistry*, 2020, vol. 28 (115816)